# EUROPEAN PATENT APPLICATION

(11) **EP 4 357 349 A1**
(43) Date of publication of application: **24.04.2024**
(21) Application number: 22824963.7
(22) Date of filing: 13.06.2022
(51) Int. Cl.: C07D 498/04, H01L 51/50

(54) **AMINE COMPOUND HAVING AZABENZOXAZOLE RING STRUCTURE, AND ORGANIC ELECTROLUMINESCENT ELEMENT USING SAME**

(30) Priority: 15.06.2021 JP 2021099695
(71) Applicant: Hodogaya Chemical Co., Ltd., Tokyo, 105-0021 (JP)
(72) Inventor: KASE, Kouki, Tokyo 105-0021 (JP); CHIBA, Eriko, Tokyo 105-0021 (JP); YANG, Byung-Sun, Tokyo 105-0021 (JP); HWANG, Moon-Chan, Tokyo 105-0021 (JP); HIRAYAMA, Yuta, Tokyo 105-0021 (JP); HAYASHI, Shuichi, Tokyo 105-0021 (JP)
(74) Representative: Forstmeyer, Dietmar
(86) International application number: PCT/JP2022/023676
(87) International publication number: WO 2022/264974

(57) **Abstract**

It is an object of the present invention is to provide a compound that, in order to prevent deterioration of the inside of an organic EL element and significantly improve the light extraction efficiency, absorbs light rays with wavelengths of 400 to 410 nm, thereby preventing the light rays from affecting a material inside the element, and has a high refractive index in a wavelength range of 450 to 750 nm. The present invention provides an amine compound having a specific benzazole ring structure having a high refractive index. In an organic EL element having at least an anode electrode, a hole transport layer, a light emitting layer, an electron transport layer, a cathode electrode, and a capping layer arranged in this order, the capping layer preferably contains the amine compound having a specific benzazole ring structure of the present invention.

## Description

### Technical Field

The present invention relates to a compound suitable for a self-luminescent electronic element favorably used in various display devices, and particularly relates to a compound suitable for an organic electroluminescent element (hereinafter abbreviated as an "organic EL element"), as well as an organic EL element, an electronic device, or an electronic element using the compound.

### Background Art

Since organic EL elements are self-luminescent elements, they are brighter, have superior display viewability, and can provide a clearer display, compared with liquid crystal elements. For these reasons, active studies have been carried out on organic EL elements.

In 1987, C. W. Tang et al. of Eastman Kodak Company produced a practical organic EL element made of an organic material, by developing an element having a stacked layer structure in which various functions were assigned to different materials. They achieved a high luminance of 1,000 cd/m² or higher at a voltage of 10V or less by stacking a layer of a fluorescent body capable of transporting electrons and a layer of an organic substance capable of transporting holes, injecting both charges into the fluorescent body layer, and thereby causing the layer to emit light (see Patent Literatures 1 and 2, for example).

Many improvements have been heretofore made to organic EL elements to put them to practical use. Due to development of electroluminescent elements in which various functions of the stacked layer structure are subdivided even further, and an anode, a hole injection layer, a hole transport layer, a light emitting layer, an electron transport layer, an electron injection layer, and a cathode are sequentially provided on a substrate, high efficiency and durability have been achieved in light emitting elements with a bottom emission structure in which light is emitted from the bottom (see Non-Patent Literature 1, for example).

Recently, light emitting elements with a top emission structure in which a metal having a high work function is used for an anode and light is emitted from the top are coming into use. Although light emitting elements with a bottom emission structure in which light is extracted from the bottom having a pixel circuit have an issue in that the area of the light emitting portion is limited, light emitting elements with a top emission structure are advantageous in that a large light emitting portion can be realized because light is extracted from the top and therefore is not blocked by the pixel circuit. In light emitting elements with a top emission structure, translucent electrodes made of LiF/Al/Ag (see Non-Patent Literature 2, for example), Ca/Mg (see Non-Patent Literature 3, for example), LiF/MgAg, or the like are used for a cathode.

In such light emitting elements, when light that is emitted by a light emitting layer is incident on another film, light rays incident at a certain angle or more are totally reflected at an interface between the light emitting layer and the other film. Thus, light that can be used has been limited to only a part of the emitted light. Recently, light emitting elements have been proposed in which a "capping layer" with a high refractive index is provided on the outside of a translucent electrode with a low refractive index, in order to improve the light extraction efficiency (see Non-Patent Literatures 2 and 3, for example).

Regarding the effect of the capping layer in light emitting elements with a top emission structure, while a light emitting element using Ir(ppy)₃ as a light emitting material has a current efficiency of 38 cd/A in the case of not having a capping layer, the current efficiency is increased to 64 cd/A by providing a ZnSe layer with a thickness of 60 nm on the light emitting element as a capping layer, which indicates that the efficiency is improved about 1.7 times. Furthermore, it is indicated that a maximum point of the transmittance and a maximum point of the efficiency of the translucent electrode and the capping layer do not absolutely match each other, and the maximum point of the light extraction efficiency is determined by interference effects (see Non-Patent Literature 3, for example).

Conventionally, it has been proposed to use a fine metal mask to form a capping layer, but this configuration is problematic in that a metal mask is deformed by heat when used at a high temperature, which deteriorates the positioning precision. For example, ZnSe has a high melting point of 1100°C or higher (see Non-Patent Literature 3, for example), and thus vapor deposition cannot be performed at an accurate position with a fine metal mask, which may affect the light emitting element itself. Furthermore, film forming through sputtering also affects the light emitting element, and thus a capping layer using an inorganic material as a constituent material cannot be suitably used.

In addition, when tris(8-hydroxyquinoline)aluminum (hereinafter abbreviated as "Alq₃") is used for a capping layer for adjusting the refractive index (see Non-Patent Literature 2, for example), since Alq₃ is known as an organic EL material that is commonly used as a green light emitting material or electron transport material, and is poor in absorption at wavelengths around 450 nm, which are used for blue light emitting materials, there is a problem in that both the color purity and the light extraction efficiency deteriorate when Alq₃ is used in blue light emitting elements.

Moreover, there also is a problem in that an element that is produced using a conventional capping layer allows light rays with wavelengths of 400 to 410 nm of sunlight to pass through, which affects the materials inside the element, resulting in a deterioration in the color purity and a deterioration in the light extraction efficiency.

In order to improve the element characteristics of an organic EL element, in particular, in order that light rays with wavelengths of 400 to 410 nm of sunlight are absorbed so as not to affect the materials inside the element, and that the light extraction efficiency is significantly improved, there is a demand for a material a capping layer, the material having a high absorption coefficient and a high refractive index, being stable in a thin film state, and having excellent durability.

### Citation List

### Patent Literature

Patent Literature 1: US 5792557A
Patent Literature 2: US 5639914A
Patent Literature 3: WO 2014/009310
Patent Literature 4: US 2014/0225100A1

### Non-Patent Literature

Non-Patent Literature 1: Proceedings of the 9th Meeting of the Japan Society of Applied Physics, pp. 55-61 (2001)
Non-Patent Literature 2: Appl. Phys. Let., 78, 544 (2001)
Non-Patent Literature 3: Appl. Phys. Let., 82, 466 (2003)
Non-Patent Literature 4: SYNLETT., 7, 1172 (2009)
Non-Patent Literature 5: J. Org. Chcm., 60, 7508 (1995)
Non-Patent Literature 6: Synth. Commun., 11, 513 (1981)
Non-Patent Literature 7: Appl. Phys. Lett., 98, 083302 (2011)

### Summary of Invention

It is an object of the present invention to provide, in order to improve the element characteristics of an organic EL element, a compound that, in particular, absorbs light rays with wavelengths of 400 to 410 nm of sunlight, thereby preventing the light rays from affecting a material inside the element, and also has a high refractive index in a wavelength range of 450 to 750 nm. It is another object of the present invention to provide an organic EL element in which deterioration of the inside of the element is suppressed and light extraction efficiency is significantly improved, by using the compound above as a constituent material for a capping layer.

A material for a capping layer suitable for an organic EL element is required to have the following physical properties: (1) a high absorption coefficient, (2) a high refractive index, (3) vapor deposition capability, (4) good stability in a thin film state, and (5) a high glass transition point. Also, an organic EL element provided by the present invention is required to have the following physical properties: (1) capability of absorbing light with wavelengths of 400 to 410 nm, (2) good light extraction efficiency, (3) no deterioration in the color purity, (4) light transmittability without change over time, and (5) a long lifespan.

Therefore, to achieve the above-described objects, the inventors of the present invention focused on the fact that arylamine-based materials have excellent stability in a thin film state and durability, selected specific materials having a high absorbance at wavelengths of 400 to 410 nm in an absorption spectrum at a concentration of 10⁻⁵ mol/L, from amine compounds having a specific benzazole ring structure having a high refractive index, then produced organic EL elements using the selected materials as constituent materials for capping layers, and thoroughly evaluated the properties of the elements. As a result, the present invention was accomplished.

That is, the present invention provides an amine compound having an azabenzoxazole ring structure below and an organic EL element using the same. Also, the present invention provides an electronic element having an organic layer containing the amine compound and an electronic device in which the electronic element is used.
1) An amine compound having an azabenzoxazole ring structure and being represented by the general formula (a-1) below.

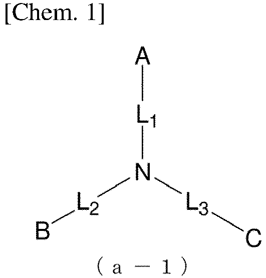

In the general formula (a-1) above, A, B, and C may be the same or different, and each represents a group represented by the general formula (b-1) below, a substituted or unsubstituted aromatic hydrocarbon group, a substituted or unsubstituted aromatic heterocyclic group, or a substituted or unsubstituted fused polycyclic aromatic group, however, at least one of A, B, and C representing a group represented by the general formula (b-1) below, and

L₁, L₂, and L₃ may be the same or different, and each represents a single bond, a substituted or unsubstituted divalent aromatic hydrocarbon group, a substituted or unsubstituted divalent aromatic heterocyclic group, or a substituted or unsubstituted divalent fused polycyclic aromatic group.

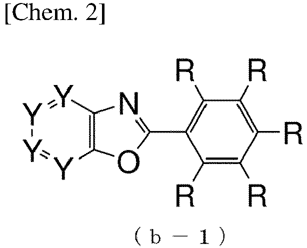

In the formula above, symbols R may be the same or different, and each represents a binding site for L₁, L₂, or L₃ in the general formula (a-1), a hydrogen atom, a deuterium atom, a chlorine atom, a cyano group, a nitro group, a trimethylsilyl group, a triphenylsilyl group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aryloxy group, a substituted or unsubstituted aromatic hydrocarbon group, a substituted or unsubstituted aromatic heterocyclic group, or a substituted or unsubstituted fused polycyclic aromatic group, and one of the symbols R represents a binding site for L₁, L₂, or L₃ in the general formula (a-1), and
symbols Y may be the same or different, and each represents a carbon atom or nitrogen atom substituted by R, however at least one of the symbols Y representing a nitrogen atom substituted by R.

2) The amine compound having an azabenzoxazole ring structure as set forth in clause 1), wherein among of A, B, and C in the general formula (a-1), a group other than the group represented by the general formula (b-1) is a substituted or unsubstituted phenyl group, a substituted or unsubstituted biphenyl group, a substituted or unsubstituted naphthyl group, a substituted or unsubstituted phenanthrenyl group, a substituted or unsubstituted carbazolyl group, a substituted or unsubstituted benzofuranyl group, a substituted or unsubstituted benzothiophenyl group, a substituted or unsubstituted indolyl group, a substituted or unsubstituted benzimidazolyl group, a substituted or unsubstituted imidazopyridyl group, a substituted or unsubstituted benzoxazolyl group, a substituted or unsubstituted benzothiazolyl group, a substituted or unsubstituted dibenzofuranyl group, or a substituted or unsubstituted dibenzothiophenyl group.

3) The amine compound having an azabenzoxazole ring structure as set forth in clause 2), wherein the group represented by the general formula (b-1) is a group represented by the general formula (b-2) or (b-3) below.

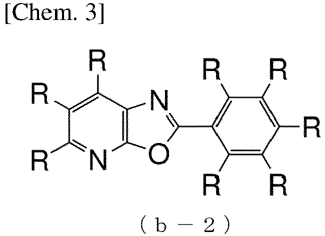

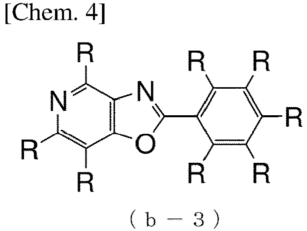

In the formulae above, symbols R in the general formulae (b-2) and (b-3) are the same as defined for the symbols R in the general formula (b-1).

4) The amine having an azabenzoxazole ring structure as set forth in clause 3), wherein the group represented by the general formula (b-2) is a group represented by the general formula (b-4) below, and the group represented by the general formula (b-3) is a group represented by the general formula (b-5) below.

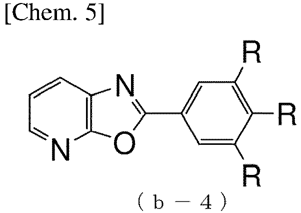

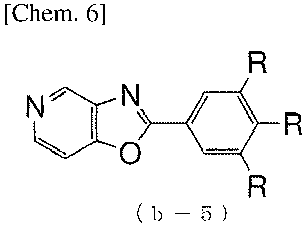

In the formulae above, symbols R in the general formulae (b-4) and (b-5) are the same as defined for the symbols R in the general formula (b-1).

5) The amine compound having an azabenzoxazole ring structure as set forth in clause 1), wherein L₁, L₂, and L₃ in the general formula (a-1) each represents a single bond, a substituted or unsubstituted phenylene group, a substituted or unsubstituted biphenylene group, a substituted or unsubstituted naphthylene group, a substituted or unsubstituted dibenzofuranylene group, or a substituted or unsubstituted dibenzothiophenylene group.

6) The amine compound having an azabenzoxazole ring structure as set forth in clause 4), wherein only one of A, B, and C in the general formula (a-1) represents a group represented by the general formula (b-4) or (b-5).

7) The amine compound having an azabenzoxazole ring structure as set forth in clause 4), wherein two of A, B, and C in the general formula (a-1) each represents a group represented by the general formula (b-4) or (b-5).

8) The amine compound having an azabenzoxazole ring structure as set forth in clause 4), wherein all of A, B, and C in the general formula (a-1) each represents a group represented by the general formula (b-4) or (b-5).

9) An organic EL element having at least an anode electrode, a hole transport layer, a light emitting layer, an electron transport layer, a cathode electrode, and a capping layer arranged in this order, wherein the capping layer contains the amine compound having an azabenzoxazole ring structure as set forth in any one of clauses 1) to 8).

10) The organic EL element as set forth in clause 9), wherein the capping layer has an extinction coefficient of 0.2 or more in a wavelength range of 400 to 410 nm, and an absorbance of 0.2 or more in a wavelength range of 400 to 410 nm in an absorption spectrum at a concentration of 10⁻⁵mol/L.

11) The organic EL element as set forth in clause 9), wherein the capping layer has a refractive index of 1.85 or more in a wavelength range of 450 to 750 nm.

12) The organic EL element as set forth in clause 9),
wherein the capping layer is
a mixed layer composed of two or more compounds including the said amine compound having an azabenzoxazole ring structure, or
a lamination layer with two or more layers wherein each layer contains the said amine compound alone respectively.

13) An electronic element having a pair of electrodes and an organic layer sandwiched therebetween, wherein the organic layer contains the amine compound having an azabenzoxazole ring structure as set forth in any one of clauses 1) to 8).

14) An electronic device in which the electronic element as set forth in clause 13) is used.

The "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by A, B, C, L₁, L₂, L₃, and R in the general formulae (a-1) and (b-1) may specifically refer to a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, an imidazopyridyl group, a benzooxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, or the like, or may specifically refer to a group selected from aryl groups having 6 to 30 carbon atoms and heteroaryl groups having 2 to 20 carbon atoms.

The "linear or branched alkyl group having 1 to 6 carbon atoms", the "cycloalkyl group having 5 to 10 carbon atoms", the "linear or branched alkenyl group having 2 to 6 carbon atoms", the "linear or branched alkyloxy group having 1 to 6 carbon atoms", the "cycloalkyloxy group having 5 to 10 carbon atoms", or the "aryloxy group" in the "linear or branched alkyl group having 1 to 6 carbon atoms and optionally having a substituent", the "cycloalkyl group having 5 to 10 carbon atoms and optionally having a substituent", the "linear or branched alkenyl group having 2 to 6 carbon atoms and optionally having a substituent", the "linear or branched alkyloxy group having 1 to 6 carbon atoms and optionally having a substituent", the "cycloalkyloxy group having 5 to 10 carbon atoms and optionally having a substituent", or the "substituted or unsubstituted aryloxy group" represented by R in the general formula (b-1) may specifically refer to a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamanthyl group, a 2-adamanthyl group, a vinyl group, an aryl group, an isopropenyl group, a 2-butenyl group, a methyloxy group, an ethyloxy group, an n-propyloxy group, a cyclopentyloxy group, a cyclohexyloxy group, a 1-adamantyloxy group, a phenyloxy group, a tolyloxy group, a biphenyloxy group, or the like.

The "substituent" in the "substituted aromatic hydrocarbon group", the "substituted aromatic heterocyclic group", the "substituted fused polycyclic aromatic group", the "linear or branched alkyl group having 1 to 6 carbon atoms and optionally having a substituent", the "cycloalkyl group having 5 to 10 carbon atoms and optionally having a substituent", the "linear or branched alkenyl group having 2 to 6 carbon atoms and optionally having a substituent", the "linear or branched alkyloxy group having 1 to 6 carbon atoms and optionally having a substituent", or the "linear or branched cycloalkyloxy group having 5 to 10 carbon atoms and optionally having a substituent" represented by A, B, C, L₁, L₂, L₃, and R in the general formulae (a-1) and (b-1) may specifically refer to a deuterium atom, a cyano group, or a nitro group; a halogen atom such as a fluorine atom, a chlorine atom, a bromine atom, or an iodine atom; a silyl group such as a trimethylsilyl group or a triphenylsilyl group; a linear or branched alkyl group having 1 to 6 carbon atoms such as a methyl group, an ethyl group, or a propyl group; a linear or branched alkyloxy group having 1 to 6 carbon atoms such as a methyloxy group, an ethyloxy group, or a propyloxy group; an alkenyl group such as a vinyl group or an aryl group; an aryloxy group such as a phenyloxy group or a tolyloxy group; an arylalkyloxy group such as a benzyloxy group or a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group; or a pyridyl group, a thienyl group, a furyl group, a pyrrolyl group, a quinolyl group, an isoquinolyl group, a benzofuranyl group, a benzothienyl group, an indolyl group, a carbazolyl group, an imidazopyridyl group, a benzooxazolyl group, a benzothiazolyl group, a quinoxalinyl group, a benzimidazolyl group, a pyrazolyl group, a dibenzofuranyl group, a dibenzothienyl group, or a carbolinyl group, or may specifically refer to an aryl group having 6 to 30 carbon atoms, a heteroaryl group having 2 to 20 carbon atoms, or the like. These substituents may be further substituted with the above-listed substituents. Furthermore, benzene rings substituted with these substituents or a plurality of substituents on the same benzene ring may be bonded to each other via a single bond, a substituted or unsubstituted methylene group, an oxygen atom, or a sulfur atom to form a ring.

In the amine compound having an azabenzoxazole ring structure of the present invention, in view of the absorption coefficient and the refractive index, L₁, L₂, and L₃ in the general formula (a-1) preferably each represents a single bond, a substituted or unsubstituted phenylene group, or a substituted or unsubstituted naphthylene group, more preferably each represents a single bond, an unsubstituted phenylene group, or an unsubstituted naphthylene group.

In the amine compound having an azabenzoxazole ring structure of the present invention, in view of the absorption coefficient and the refractive index, preferably, one of the symbols R in each of the general formulae (b-4) and (b-5) represents a binding site for L₁, L₂, or L₃ in the general formula (a-1), and the remaining two represent hydrogen atoms.

In view of the stability in a thin film state, the amine compound having an azabenzoxazole ring structure of the present invention preferably has a glass transition point of 100°C or higher.

In the organic EL element of the present invention, in view of the light extraction efficiency, the capping layer preferably has a thickness in a range of 30 to 120 nm, more preferably in a range of 40 to 80 nm.

In the organic EL element of the present invention, from the viewpoint of suppressing deterioration of the inside of the element, it is preferable that the capping layer has an extinction coefficient of 0.2 or more in a wavelength range of 400 to 410 nm, and an absorbance of 0.2 or more in a wavelength range of 400 to 410 nm in an absorption spectrum at a concentration of 10⁻⁵mol/L. The extinction coefficient is more preferably 0.5 or more, and the absorbance is more preferably 0.3 or more.

In the organic EL element of the present invention, in view of the light extraction efficiency, the capping layer preferably has a refractive index of 1.85 or more, more preferably 1.90 or more, in a wavelength range of 450 to 750 nm.

In the organic EL element of the present invention, the capping layer may be formed as a mixed layer composed of two or more compounds or a stacked layer in which layers each containing respective one of the two or more compounds alone are stacked. However, at least one of the two or more compounds is the amine compound having an azabenzoxazole ring structure of the present invention.

The amine compound having an azabenzoxazole ring structure and being represented by the general formula (a-1) of the present invention has the following features: 1) a high absorption coefficient, (2) a high refractive index in a wavelength range of 450 to 750 nm, (3) vapor deposition capability, (4) good stability in a thin film state, and (5) high heat resistance. By using the compound of the present invention as a material of the capping layer that is provided outside the transparent or translucent electrode of the organic EL element and has a higher refractive index than the translucent electrode, it is possible to obtain an organic EL element that can achieve significantly improved light extraction efficiency and in which deterioration of materials inside the element is suppressed.

### Brief Description of Drawings

[Fig. 1] Fig. 1 shows the structures of Compounds (1) to (12) as examples of the compound of the present invention.
[Fig. 2] Fig. 2 shows the structures of Compounds (13) to (27) as examples of the compound of the present invention.
[Fig. 3] Fig. 3 shows the structures of Compounds (28) to (42) as examples of the compound of the present invention.
[Fig. 4] Fig. 4 shows the structures of Compounds (43) to (54) as examples of the compound of the present invention.
[Fig. 5] Fig. 5 shows the structures of Compounds (55) to (66) as examples of the compound of the present invention.
[Fig. 6] Fig. 6 shows the structures of Compounds (67) to (81) as examples of the compound of the present invention.
[Fig. 7] Fig. 7 shows the structures of Compounds (82) to (96) as examples of the compound of the present invention.
[Fig. 8] Fig. 8 shows the structures of Compounds (97) to (111) as examples of the compound of the present invention.
[Fig. 9] Fig. 9 shows the structures of Compounds (112) to (126) as examples of the compound of the present invention.
[Fig. 10]Fig. 10 shows the structures of Compounds (127) to (138) as examples of the compound of the present invention.
[Fig. 11] Fig. 11 shows the structures of Compounds (139) to (153) as examples of the compound of the present invention.
[Fig. 12]Fig. 12 shows the structures of Compounds (154) to (165) as examples of the compound of the present invention.
[Fig. 13]Fig. 13 shows the structures of Compounds (166) to (173) as examples of the compound of the present invention.
[Fig. 14]Fig. 14 shows the structure of organic EL elements of examples and comparative examples of the present invention.

### Description of Embodiments

Amine compounds having an azabenzoxazole ring structure and being represented by the general formula (a-1) of the present invention are novel compounds, and as described below, an azabenzoxazole derivative serving as the main backbone of these compounds can be synthesized using a method that is known per se (see Non-Patent Literature 4, for example). Furthermore, an amine compound having an azabenzoxazole ring structure and being represented by the general formula (a-1) of the present invention can be synthesized by subjecting a synthesized halogenated azabenzoxazole derivative and an arylamine to a coupling reaction with a copper catalyst, a palladium catalyst, or the like. In addition, an amine compound having an azabenzoxazole ring structure and being represented by the general formula (a-1) of the present invention can be synthesized similarly by converting a halogenated azabenzoxazole derivative to a boronic acid ester derivative and then subjecting the boronic acid ester derivative and a halogenated arylamine to a coupling reaction (see Non-Patent Literature 5 and Non-Patent Literature 6, for example).

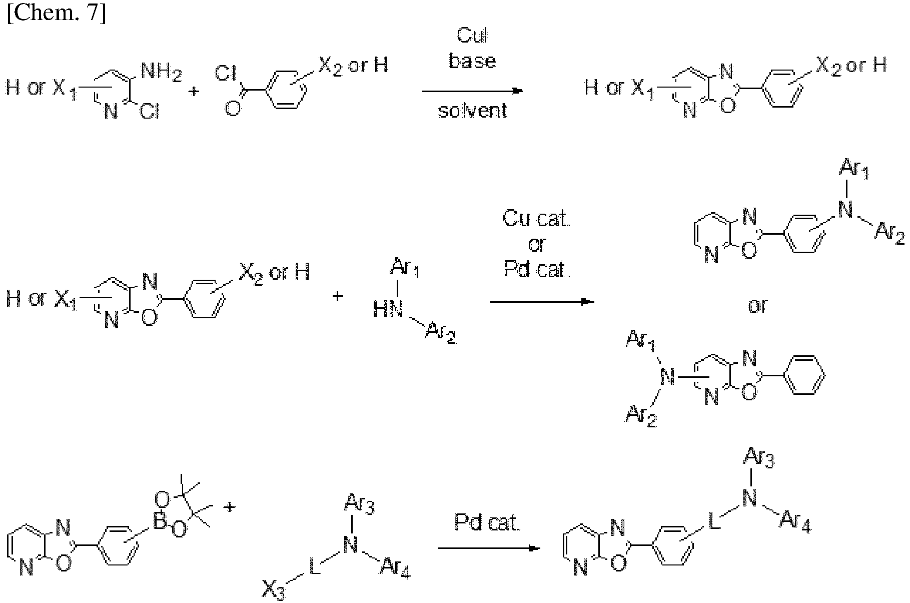

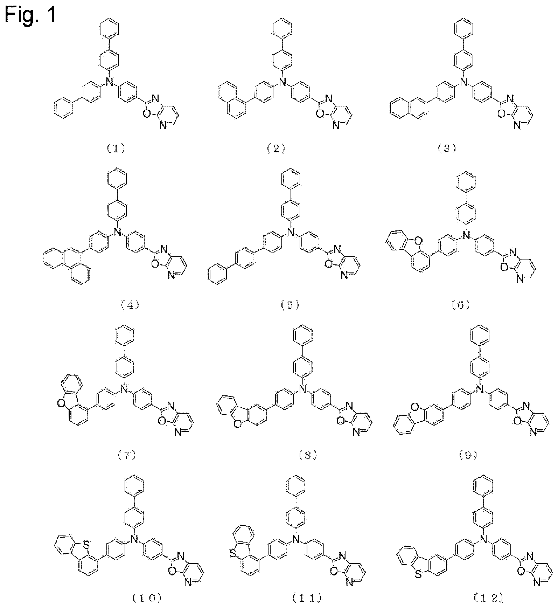

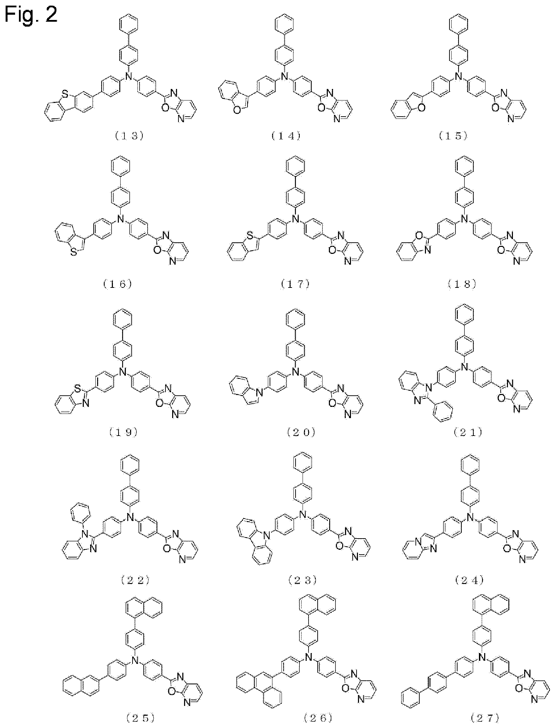

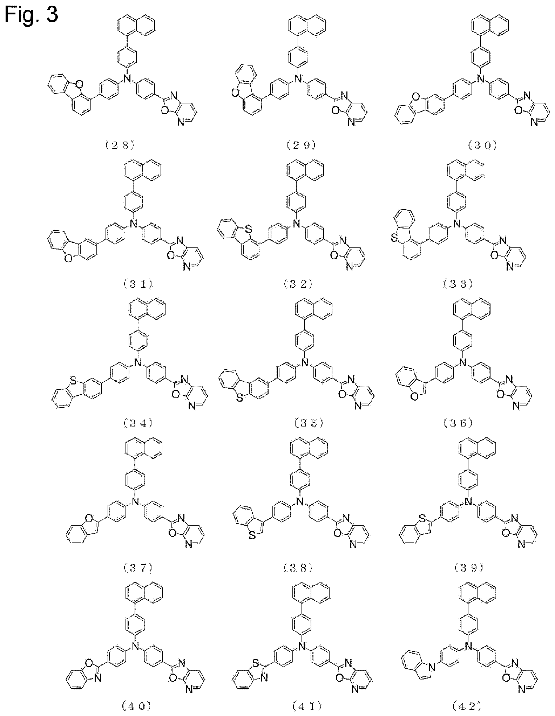

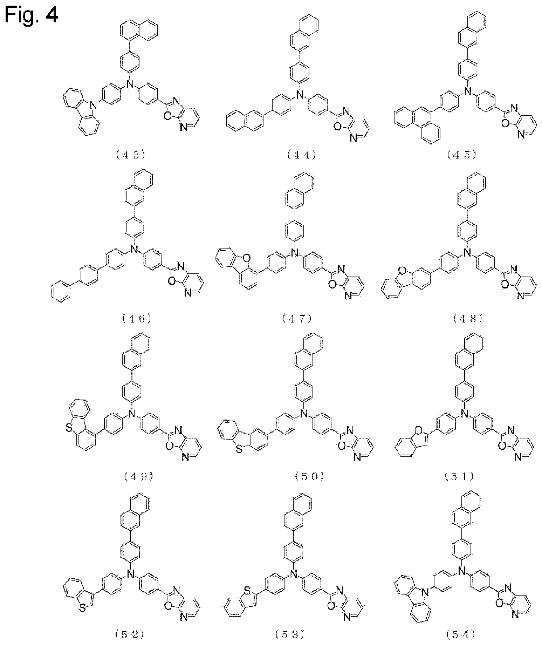

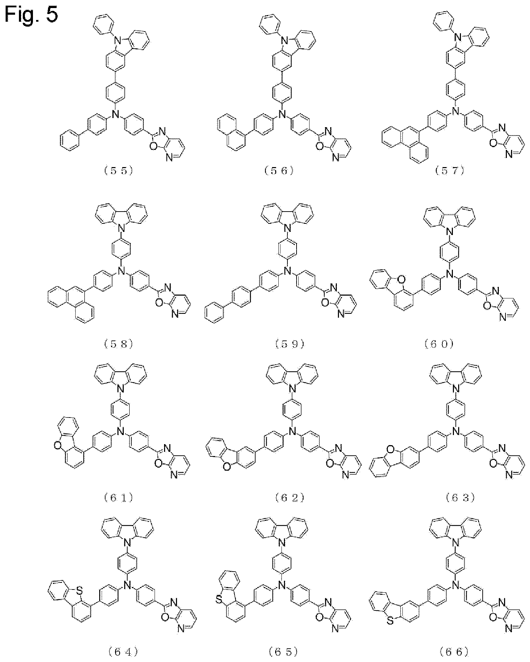

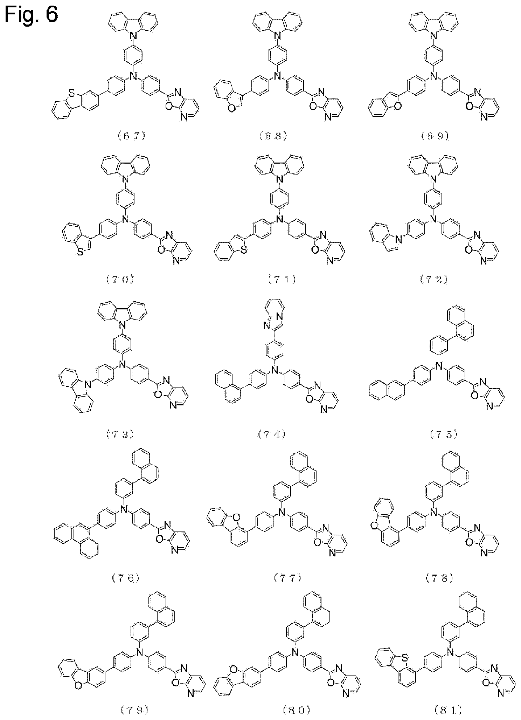

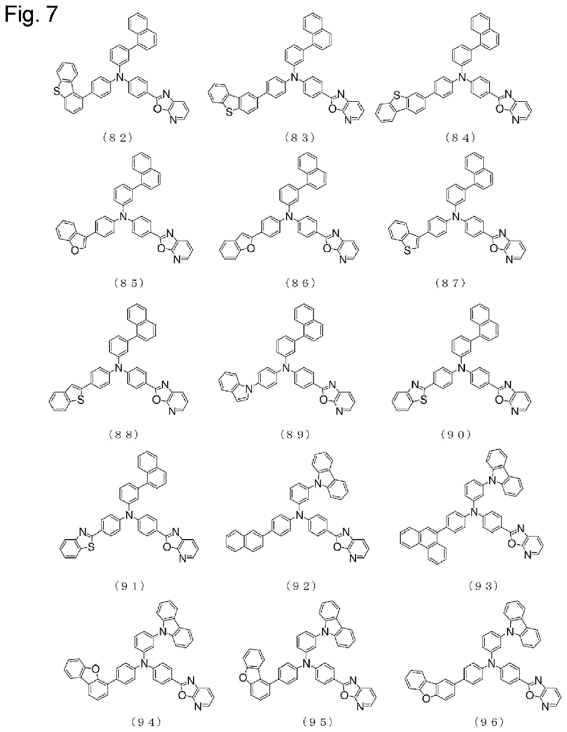

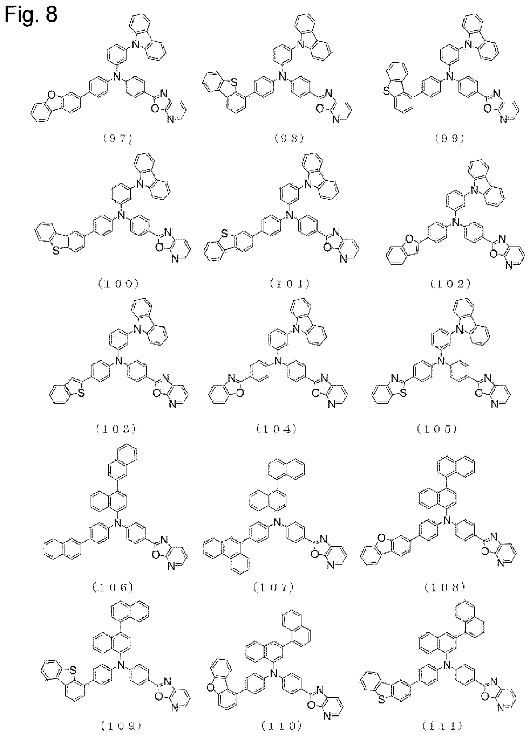

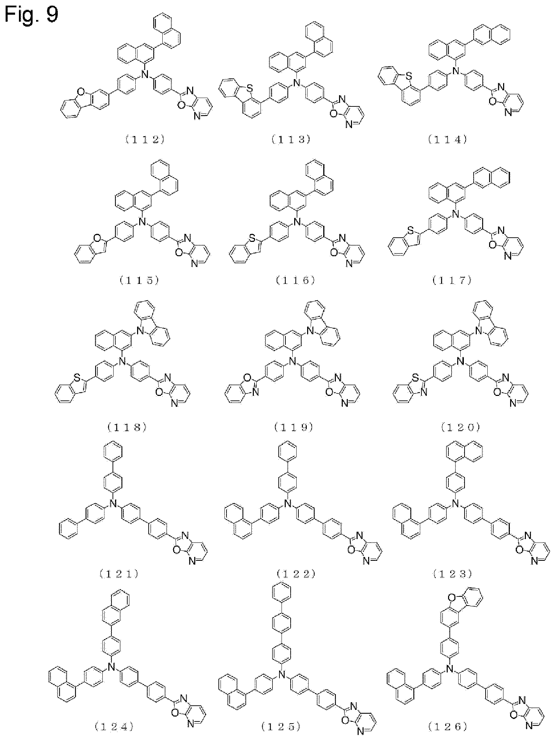

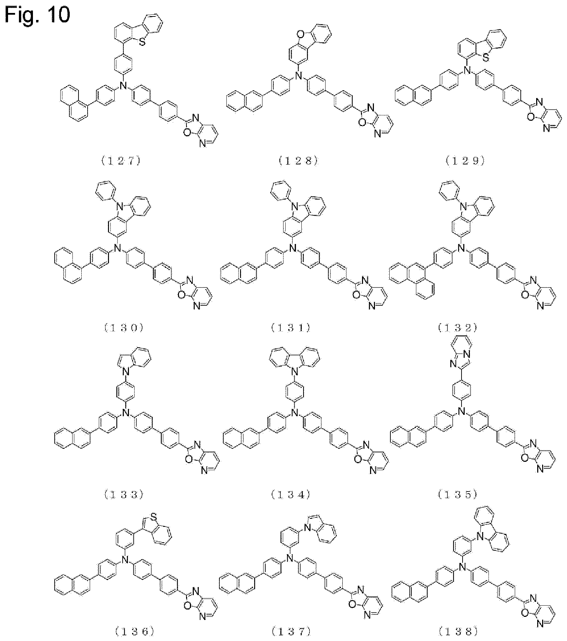

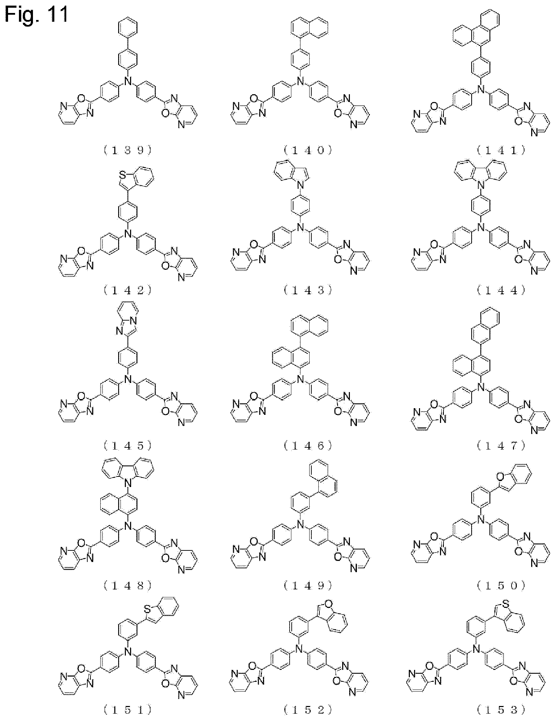

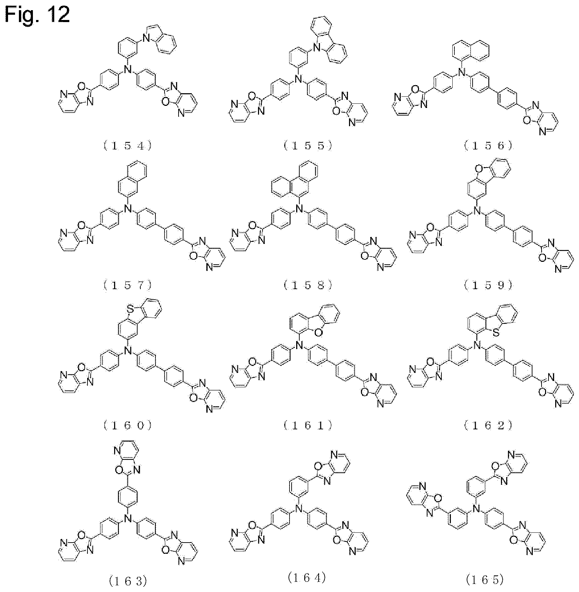

Figs. 1 to 13 show specific examples of preferred amine compounds having an azabenzoxazole ring structure and being represented by the general formula (a-1) above, suitable for use in an organic EL element of the present invention, but the invention is not limited to these compounds.

These is no particular limitation on the method for purifying an amine compound having an azabenzoxazole ring structure and being represented by the general formula (a-1) above, but the amine compound can be purified using a known method used for purification of organic compounds, such as purification by column chromatography, adsorption purification using silica gel, activated carbon, activated clay, or the like, recrystallization or crystallization purification using a solvent, and sublimation purification. Compounds can be identified by NMR analysis. Preferably, the melting point, the glass transition point (Tg), and the refractive index are measured as physical properties. The melting point is an indicator of vapor deposition properties, the glass transition point (Tg) is an indicator of stability in a thin film state, and the refractive index is an indicator of the improvement of the light extraction efficiency.

The melting point and the glass transition point (Tg) can be measured using powder with a high-sensitivity differential scanning calorimeter (DSC3100SA manufactured by Bruker AXS K.K.).

The refractive index and the extinction coefficient can be obtained by performing measurement on an 80-nm thin film formed on a silicon substrate, using a spectroscopic measurement device (F10-RT-UV manufactured by Filmetrics).

The absorbance can be measured using a solution adjusted to a concentration of 10⁻⁵ mol/L using a toluene solvent, and the absorption coefficient can be measured using solutions adjusted to four different concentrations of 5.0×10⁻⁶ mol/L, 1.0×10⁻⁵ mol/L, 1.5×10⁻⁵ mol/L, and 2.0×10⁻⁵ mol/L using a toluene solvent, with use of a UV-NIR spectrophotometer (V-650 manufactured by JASCO Corporation).

The organic EL element of the present invention for use as a light emitting element with a top emission structure may have a structure constituted by an anode, a hole transport layer, a light emitting layer, an electron transport layer, a cathode, and a capping layer sequentially provided on a glass substrate, a structure in which a hole injection layer is further provided between the anode and the hole transport layer, a structure in which an electron blocking layer is further provided between the hole transport layer and the light emitting layer, a structure in which a hole blocking layer is further provided between the light emitting layer and the electron transport layer, and a structure in which a an electron injection layer is further provided between the electron transport layer and the cathode. In these multilayer structures, a single organic layer may have the functions of a plurality of layers, and, for example, it is possible to adopt a configuration in which an organic layer serves as both the hole injection layer and the hole transport layer, a configuration in which an organic layer serves as both the hole transport layer and the electron blocking layer, a configuration in which an organic layer serves as both the hole blocking layer and the electron transport layer, a configuration in which an organic layer serves as both the electron transport layer and the electron injection layer, and the like. Furthermore, two or more organic layers having the same function may be stacked, and, for example, it is possible to adopt a configuration in which two hole transport layers are stacked, a configuration in which two light emitting layers are stacked, a configuration in which two electron transport layers are stacked, a configuration in which two capping layers are stacked, and the like.

The total film thickness of the layers of the organic EL element is preferably approximately from 200 to 750 nm, and more preferably approximately from 350 to 600 nm. Furthermore, the film thickness of the capping layer is, for example, preferably from 30 to 120 nm, and more preferably from 40 to 80 nm. In this case, it is possible to obtain good light extraction efficiency. Note that the film thickness of the capping layer may be changed as appropriate according to the type of light emitting material used in the light emitting element, the thickness of the organic EL element excluding the capping layer, and the like.

An electrode material having a high work function, such as ITO or gold, is used for the anode of the organic EL element of the present invention.

Arylamine compounds having three or more triphenylamine structures in the molecule and having a structure in which these triphenylamine structures are linked to each other via a single bond or a divalent group that does not contain a heteroatom, for example, starburst triphenylamine derivatives, various triphenylamine tetramers, and other materials can be used for the hole injection layer of the organic EL element of the present invention. In addition, porphyrin compounds typified by copper phthalocyanine, acceptor type heterocyclic compounds such as hexacyanoazatriphenylene, and coating type polymer materials can also be used. The hole injection layer may be formed using one of the above-listed materials alone, or a single layer that is formed using a mixture with another material may be used as the hole injection layer. Alternatively, the hole injection layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked. With these materials, a thin film can be formed using vapor deposition, or another known method such as spin coating or inkjet printing.

Benzidine derivatives such as N,N'-diphenyl-N,N'-di(m-tolyl)benzidine (hereinafter abbreviated as "TPD"), N,N'-diphenyl-N,N'-di(α-naphthyl)benzidine (hereinafter abbreviated as "NPD"), and N,N,N',N'-tetrabiphenylyl benzidine; 1,1-bis[4-(di-4-tolylamino)phenyl]cyclohexane (hereinafter abbreviated as "TAPC"); and the like can be used for the hole transport layer of the organic EL element of the present invention. In particular, arylamine compounds having two triphenylamine structures in the molecule and having a structure in which these triphenylamine structures are linked to each other via a single bond or a divalent group that does not contain a heteroatom, for example, N,N,N',N'-tetrabiphenylyl benzidine and the like are preferably used. Also, arylamine compounds having three or more triphenylamine structures in the molecule and having a structure in which these triphenylamine structures are linked to each other via a single bond or a divalent group that does not contain a heteroatom, for example, various triphenylamine trimers and tetramers and the like are preferably used. The hole transport layer may be formed using one of the above-listed materials alone, or a single layer that is formed using a mixture with another material may be used as the hole transport layer. Alternatively, the hole transport layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked. Furthermore, coating type polymer materials such as poly(3,4-ethylenedioxythiophene) (hereinafter abbreviated as "PEDOT") and poly(styrenesulfonate) (hereinafter abbreviated as "PSS") can be used for the hole injection and transport layers. With these materials, a thin film can be formed using vapor deposition, or another known method such as spin coating or inkjet printing.

Furthermore, a material that is obtained by p-doping a material normally used for the hole injection layer or the hole transport layer with trisbromophenylamine hexachloroantimony, a radialene derivative (see Patent Literature 3, for example), or the like; a polymer compound that has the structure of a benzidine derivative, such as TPD, in a partial structure thereof; and the like can be used for the hole injection layer or the hole transport layer.

Compounds having an electron blocking effect, such as carbazole derivatives such as 4,4',4"-tri(N-carbazolyl)triphenylamine (hereinafter abbreviated as "TCTA"), 9,9-bis[4-(carbazol-9-yl)phenyl]fluorene, 1,3-bis(carbazol-9-yl)benzene (hereinafter abbreviated as "mCP"), and 2,2-bis(4-carbazol-9-yl-phenyl)adamantane (hereinafter abbreviated as "Ad-Cz"); compounds that have a triphenylsilyl group and a triarylamine structure and are typified by 9-[4-(carbazol-9-yl)phenyl]-9-[4-(triphenylsilyl)phenyl]-9H-fluorene; and the like, can be used for the electron blocking layer of the organic EL element of the present invention. The electron blocking layer may be formed using one of the above-listed materials alone, or a single layer that is formed using a mixture with another material may be used as the electron blocking layer. Alternatively, the electron blocking layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked. With these materials, a thin film can be formed using vapor deposition, or another known method such as spin coating or inkjet printing.

Metal complexes of quinolinol derivatives such as Alq₃, various other types of metal complexes, an anthracene derivative, a bisstyrylbenzene derivative, a pyrene derivative, an oxazole derivative, a poly(p-phenylene vinylene) derivative, and the like can be used for the light emitting layer of the organic EL element of the present invention. Moreover, the light emitting layer may also be formed using a host material and a dopant material. As the host material, an anthracene derivative is preferably used, and heterocyclic compounds having an indole ring as a partial structure of a fused ring, heterocyclic compounds having a carbazole ring as a partial structure of a fused ring, a carbazole derivative, a thiazole derivative, a benzimidazole derivative, a polydialkylfluorene derivative, and the like can be used in addition to the above-listed light emitting materials. As the dopant material, quinacridone, coumarin, rubrene, perylene, and derivatives thereof; a benzopyran derivative; a rhodamine derivative; an aminostyryl derivative; and the like can be used. The light emitting layer may be formed using one of the above-listed materials alone, or a single layer that is formed using a mixture with another material may be used as the light emitting layer. Alternatively, the light emitting layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked.

Furthermore, it is also possible to use a phosphorescent emitter as the light emitting material. As the phosphorescent emitter, a phosphorescent emitter of a metal complex of iridium, platinum, or the like can be used, and a green phosphorescent emitter such as Ir(ppy)₃, a blue phosphorescent emitter such as FIrpic or FIr6, a red phosphorescent emitter such as Btp₂Ir (acac), and the like can be used. In this case, carbazole derivatives such as 4,4'-di(N-carbazolyl)biphenyl (hereinafter abbreviated as "CBP"), TCTA, and mCP, which are host materials having hole injectability and transportability, can be used as the host material. Furthermore, p-bis(triphenylsilyl)benzene (hereinafter abbreviated as "UGH2"), 2,2',2"-(1,3,5-phenylene)-tris(1-phenyl-1H-benzimidazole) (hereinafter abbreviated as "TPBI"), and the like, which are host materials having electron transportability, can be used as the host material, and such materials enable the production of a high-performance organic EL element.

When doping the host material with a phosphorescent light emitting material, in order to avoid concentration quenching, it is preferable that the amount of the phosphorescent light emitting material is within a range of 1 to 30 wt% with respect to the entire light emitting layer and the doping is performed through co-deposition.

As the light emitting material, materials that emit delayed fluorescence, such as CDCB derivatives such as PIC-TRZ, CC2TA, PXZ-TRZ, and 4CzIPN, can also be used (see Non-Patent Literature 7, for example). With these materials, a thin film can be formed using vapor deposition, or another known method such as spin coating or inkjet printing.

Compounds having a hole blocking effect, such as a phenanthroline derivative such as bathocuproine (hereinafter abbreviated as "BCP"), a metal complex of a quinolinol derivative, such as aluminum(III)bis(2-methyl-8-quinolinato)-4-phenylphenolate (hereinafter abbreviated as "BAlq"), various types of rare-earth complexes, a triazole derivative, a triazine derivative, a pyrimidine derivative, an oxadiazole derivative, a benzazole derivative, and the like, can be used for the hole blocking layer of the organic EL element of the present invention. These materials may also serve as the material for the electron transport layer. The hole blocking layer may be formed using one of the above-listed materials alone, or a single layer that is formed using a mixture with another material may be used as the hole blocking layer. Alternatively, the hole blocking layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked. With these materials, a thin film can be formed using vapor deposition, or another known method such as spin coating or inkjet printing.

Metal complexes of quinolinol derivatives such as Alq₃ and BAlq, various other types of metal complexes, a triazole derivative, a triazine derivative, a pyrimidine derivative, an oxadiazole derivative, a pyridine derivative, a benzimidazole derivative, a benzazole derivative, a thiadiazole derivative, an anthracene derivative, a carbodiimide derivative, a quinoxaline derivative, a pyridoindole derivative, a phenanthroline derivative, a silole derivative, and the like can be used for the electron transport layer of the organic EL element of the present invention. The electron transport layer may be formed using one of the above-listed materials alone, or a single layer that is formed using a mixture with another material may be used as the electron transport layer. Alternatively, the electron transport layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked. With these materials, a thin film can be formed using vapor deposition, or another known method such as spin coating or inkjet printing.

Alkali metal salts such as lithium fluoride and cesium fluoride, alkaline earth metal salts such as magnesium fluoride, metal complexes of quinolinol derivatives such as lithium quinolinol, metal oxides such as aluminum oxide, metals such as ytterbium (Yb), samarium (Sm), calcium (Ca), strontium (Sr), and cesium (Cs), and the like can be used for the electron injection layer of the organic EL element of the present invention. When an electron transport layer and a cathode are suitably selected, the electron injection layer can be omitted.

Furthermore, a material obtained by n-doping a material normally used for the electron injection layer or the electron transport layer with a metal such as cesium can be used for the electron injection layer or the electron transport layer.

An electrode material having a low work function such as aluminum; an alloy having an even lower work function such as a magnesium-silver alloy, a magnesium-calcium alloy, a magnesium-indium alloy, or an aluminum-magnesium alloy; ITO, IZO, or the like is used as the electrode material of the cathode of the organic EL element of the present invention.

Amine compounds having an azabenzoxazole ring structure and being represented by the general formula (a-1) above are used for the capping layer of the organic EL element of the present invention. The capping layer may be formed using one of the above-listed materials alone, or a single layer that is formed using a mixture with another material may be used as the capping layer. Alternatively, the capping layer may have a structure in which layers that are each formed using one of the above-listed materials alone are stacked; a structure in which layers that are each formed using a mixture of two or more of the above-listed materials are stacked; or a structure in which a layer that is formed using one of the above-listed materials alone and a layer that is formed using a mixture of two or more of the above-listed materials are stacked. With these materials, a thin film can be formed using vapor deposition, or another known method such as spin coating or inkjet printing.

In the description above, an organic EL element with a top emission structure has been described, but the present invention is not limited thereto, and can also be applied in a similar manner to an organic EL element with a bottom emission structure or an organic EL element with a dual emission structure in which light is emitted from both of the top and the bottom. In these cases, an electrode located in a direction in which light is extracted from the light emitting element to the outside has to be transparent or translucent.

Preferably, the refractive index of the material constituting the capping layer is greater than the refractive index of an electrode located adjacent to the capping layer. That is to say, the capping layer improves the light extraction efficiency of the organic EL element, and this effect is more effective when the reflectance at an interface between the capping layer and a material that is in contact with the capping layer is greater, because the effect of optical interference is greater. Therefore, the refractive index of the material constituting the capping layer is preferably greater than the refractive index of the electrode located adjacent to the capping layer. It is sufficient that the refractive index of the capping layer is 1.70 or more, but the refractive index of the capping layer is more preferably 1.80 or more, or even more preferably 1.85 or more.

### Examples

Hereinafter, embodiments of the present invention will be described in greater detail using examples, but the present invention is not limited to the examples below and includes other matter that does not depart from the gist of the present invention.

### Example 1

### <Synthesis of bis(4-naphthalen-1-yl-phenyl)4'-(7-azabenzoxazol-2-yl)-biphenyl-4-yl-amine: Compound (123)>

5.0 g of bis(4-(naphthalen-1-yl)phenyl)-4-bromophenyl-amine, 3.1 g of 2-{4-(4,4,5,5-tetramethyl-[1,3,2]dioxaborolan-2-yl)-phenyl}-7-azabenzoxazole, 0.5 g of tetrakis(triphenylphosphine)palladium(0), and 1.8 g of potassium carbonate were placed in a nitrogen-purged reaction vessel and stirred in a toluene/EtOH/H₂O mixed solvent overnight under reflux. The reaction system was allowed to cool. Then, methanol/H₂O was added thereto, and a solid was removed by filtration. Thus, a crude product was obtained. The obtained crude product was purified through crystallization using a monochlorobenzene/acetone mixed solvent to obtain 4.0 g (with a yield of 66.7%) of an yellow powder of bis(4-naphthalen-1-yl-phenyl)4'-(7-azabenzoxazol-2-yl)-biphenyl-4-yl-amine: Compound (123).

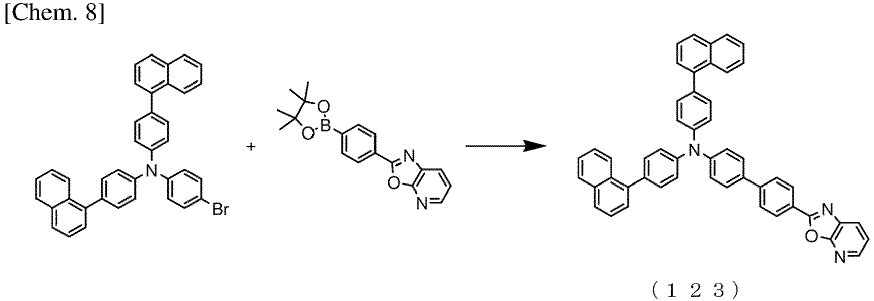

The structure of the obtained yellow powder was identified using NMR.

In ¹H-NMR (CDCl₃), the following signals of 33 hydrogens were detected.
δ (ppm) = 8.36 (3H), 8.06 (3H), 7.92 (2H), 7.86 (2H), 7.81 (2H), 7.66 (2H), 7.57-7.45 (12H), 7.41-7.33 (7H).

### Example 2

### <Synthesis of 4'-(7-azabenzoxazol-2-yl)-biphenyl-4-yl-(4-naphthalen-1-yl-phenyl)-(4-naphthalen-2-yl-phenyl)-amine: Compound (124)>

5.0 g of 4-bromophenyl-(4-naphthalen-1-yl-phenyl)-(4-naphthalen-2-yl-phenyl)-amine, 3.1 g of 2-{4-(4,4,5,5-tetramethyl-[1,3,2]dioxaborolan-2-yl)-phenyl}-7-azabenzoxazole, 0.5 g of tetrakis(triphenylphosphine)palladium(0), and 1.8 g of potassium carbonate were placed in a nitrogen-purged reaction vessel and stirred in a toluene/EtOH/H₂O mixed solvent overnight under reflux. The reaction system was allowed to cool. Then, methanol/H₂O was added thereto, and a solid was removed by filtration. Thus, a crude product was obtained. The obtained crude product was purified through crystallization using a monochlorobenzene/acetone mixed solvent to obtain 4.4 g (with a yield of 73.3%) of an yellow powder of 4'-(7-azabenzoxazol-2-yl)-biphenyl-4-yl-(4-naphthalen-1-yl-phenyl)-(4-naphthalen-2-yl-phenyl)-amine: Compound (124).

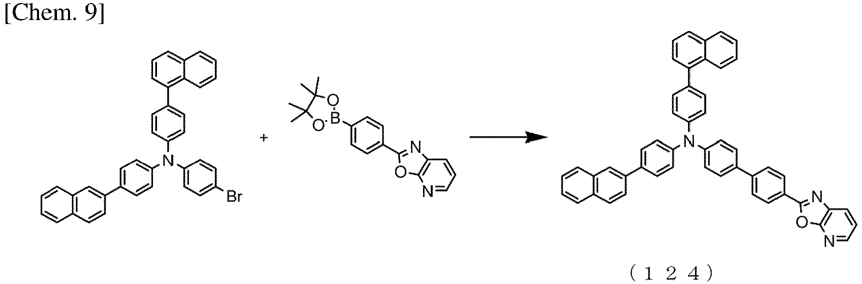

The structure of the obtained yellow powder was identified using NMR.

In ¹H-NMR (CDCl₃), the following signals of 33 hydrogens were detected.
δ (ppm) = 8.35 (3H), 8.06 (3H), 7.91 (3H), 7.86 (2H), 7.80 (2H), 7.77 (1H), 7.71 (2H), 7.64 (2H), 7.56-7.43 (8H), 7.38-7.31 (7H).

### Example 3

### <Synthesis of 4-(7-azabenzoxazol-2-yl)-phenyl-4-yl-(4-naphthalen-1-yl-phenyl)-(4-naphthalen-2-yl-phe nyl)-amine: Compound (25)>

7.0 g of (4-naphthalen-1-yl-phenyl)-(4-naphthalen-2-yl-phenyl)-amine, 5.0 g of 4-bromo-(7-azabenzoxazol-2-yl)-benzene, 0.1 g of palladium(II) acetate, 0.2 g of tri-t-butylphosphine, and 2.4 g of sodium t-butoxide were placed in a nitrogen-purged reaction vessel and stirred in a toluene solvent for 5 hours under reflux. The reaction system was allowed to cool and then dispersed and washed with methanol added thereto, and a solid was removed by filtration. Thus, a crude product was obtained. The obtained crude product was purified through crystallization using a monochlorobenzene/acetone mixed solvent to obtain 7.1 g (with a yield of 69.5%) of an yellow powder of 4-(7-azabenzoxazol-2-yl)-phenyl-(4-naphthalen-1-yl-phenyl)-(4-naphthalen-2-yl-phenyl)-amine: Compound (25).

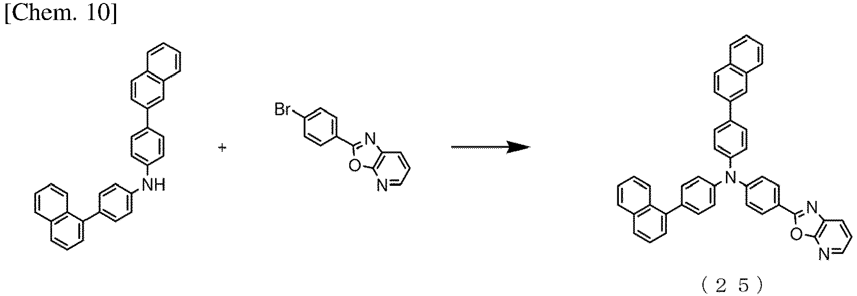

The structure of the obtained yellow powder was identified using NMR.

In ¹H-NMR (CDCl₃), the following signals of 29 hydrogens were detected.
δ (ppm) = 8.30 (1H), 8.19 (2H), 8.00 (1H), 8.05-8.01 (2H), 7.95-7.87 (5H), 7.79-7.74 (3H), 7.57-7.47 (8H), 7.40-7.30 (7H).

### Example 4

### <Synthesis of bis(4-naphthalen-2-yl-phenyl)-4-(7-azabenzoxazol-2-yl)-phenyl-amine: Compound (44) >

5.0 g of bis(4-naphthalen-2-yl-phenyl)-amine, 3.6 g of 4-bromo-(7-azabenzoxazol-2-yl)-benzene, 0.4 g of tris(dibenzylideneacetone)dipalladium(0), 0.4 g of tri-t-butylphosphine, and 1.7 g of sodium t-butoxide were placed in a nitrogen-purged reaction vessel and stirred in a xylene solvent for overnight under reflux. The reaction system was allowed to cool and then dispersed and washed with methanol added thereto, and a solid was removed by filtration. Thus, a crude product was obtained. The obtained crude product was purified through recrystallization using a monochlorobenzene to obtain 3.2 g (with a yield of 43.8%) of an yellow powder of bis(4-naphthalen-2-yl-phenyl)-4-(7-azabenzoxazol-2-yl)-phenyl-amine: Compound (44).

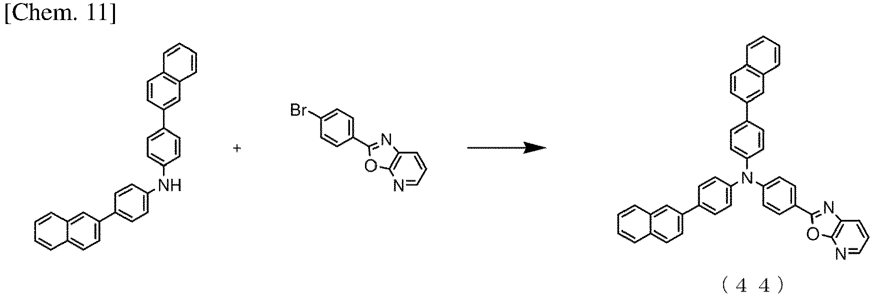

The structure of the obtained yellow powder was identified using NMR.

In ¹H-NMR (CDCl₃), the following signals of 29 hydrogens were detected.
δ (ppm) = 8.30 (1H), 8.17 (2H), 8.07 (2H), 8.02 (1H), 7.95-7.87 (6H), 7.78 (2H), 7.73 (4H), 7.54-7.47 (4H), 7.37-7.27 (7H).

### Example 5

### <Synthesis of bis(4-(7-azabenzoxazol-2-yl)-phenyl)-(4-naphthalen-1-yl-phenyl)-amine: Compound (140) >

1.9 g of 4-(naphthalen-1-yl)-phenyl-amine, 5.2 g of 4-bromo-(7-azabenzoxazol-2-yl)-benzene, 0.2 g of tris(dibenzylideneacetone)dipalladium(0), 0.2 g of tri-t-butylphosphine, and 1.9 g of sodium t-butoxide were placed in a nitrogen-purged reaction vessel and stirred in a toluene solvent for 8 hours under reflux. The reaction system was allowed to cool and then dispersed and washed with methanol added thereto, and a solid was removed by filtration. Thus, a crude product was obtained. The obtained crude product was purified through crystallization using a monochlorobenzene/acetone mixed solvent to obtain 3.8 g (with a yield of 72.1%) of an yellow powder of bis(4-(7-azabenzoxazol-2-yl)-phenyl)-(4-naphthalen-1-yl-phenyl)-amine: Compound (140).

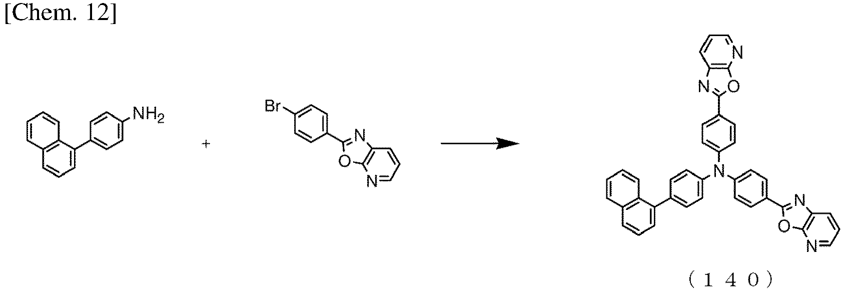

The structure of the obtained yellow powder was identified using NMR.

In ¹H-NMR (CDCl₃), the following signals of 25 hydrogens were detected.
δ (ppm) =8.33 (2H), 8.25 (4H), 8.06-8.01 (3H), 7.95-7.88 (2H), 7.58-7.49 (6H), 7.39-7.34 (8H).

### Example 6

### < Synthesis of bis(4-(7-azabenzoxazol-2-yl)-phenyl)-(4-carbazol-9-yl-phenyl)-amine: Compound (144) >

2.3 g of 4-(carbazol-9-yl)-phenyl-amine, 5.1 g of 4-bromo-(7-azabenzoxazol-2-yl)-benzene, 0.2 g of tris(dibenzylideneacetone)dipalladium(0), 0.2 g of tri-t-butylphosphine, and 2.0 g of sodium t-butoxide were placed in a nitrogen-purged reaction vessel and stirred in a toluene solvent for 6 hours under reflux. The reaction system was allowed to cool and then dispersed and washed with methanol added thereto, and a solid was removed by filtration. Thus, a crude product was obtained. The obtained crude product was purified through crystallization using a monochlorobenzene/acetone mixed solvent to obtain 3.9 g (with a yield of 67.7%) of an yellow powder of bis(4-(7-azabenzoxazol-2-yl)-phenyl)-(4-carbazol-9-yl-phenyl)-amine: Compound (144).

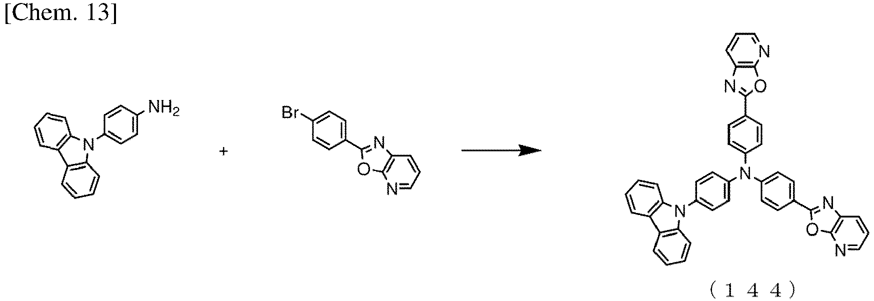

The structure of the obtained yellow powder was identified using NMR.

In ¹H-NMR (CDCl₃), the following signals of 26 hydrogens were detected.
δ (ppm) = 8.34 (2H), 8.27 (4H), 8.17 (2H), 8.06 (2H), 7.60 (2H), 7.52 (2H), 7.46 (4H), 7.40-7.30 (8H).

### Example 7

### <Synthesis of bis(4-(7-azabenzoxazol-2-yl)-phenyl)-[1,1']binaphthalenyl-4-yl-amine: Compound (146) >

5.0 g of [1,1']binaphthalenyl-4-yl-amine, 11.2 g of 4-bromo-(7-azabenzoxazol-2-yl)-benzene, 0.5 g of tris(dibenzylideneacetone)dipalladium(0), 0.6 g of tri-t-butylphosphine, and 5.4 g of sodium t-butoxide were placed in a nitrogen-purged reaction vessel and stirred in a toluene solvent overnight under reflux. The reaction system was allowed to cool and then dispersed and washed with methanol added thereto, and a solid was removed by filtration. Thus, a crude product was obtained. The obtained crude product was purified through crystallization using a tetrahydrofuran/acetone mixed solvent to obtain 8.2 g (with a yield of 67.2%) of an yellow powder of bis(4-(7-azabenzoxazol-2-yl)-phenyl)-[1,1']binaphthalenyl-4-yl-amine: Compound (146).

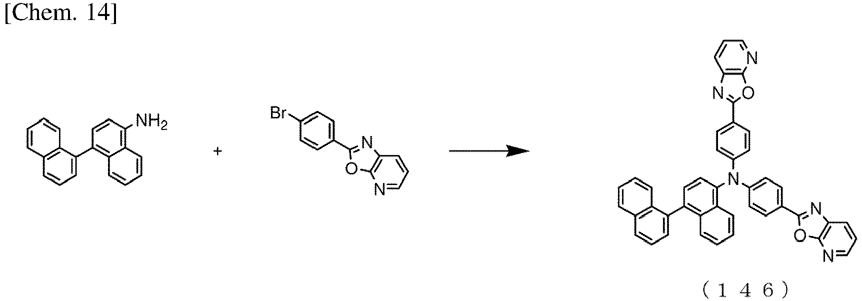

The structure of the obtained yellow powder was identified using NMR.

In ¹H-NMR (CDCl₃), the following signals of 27 hydrogens were detected.
δ (ppm) = 8.32 (2H), 8.22 (4H), 8.05-7.98 (5H), 7.64 (1H), 7.60-7.31 (15H).

### Example 8

### <Synthesis of 4-(7-azabenzoxazol-2-yl)-phenyl-(4'-(naphthalen-1-yl)-biphenyl-4-yl)-(4-(benzofuran-2-yl)-phenyl)-amine: Compound (166)>

10.0 g of (4'-(naphthalen-1-yl)-biphenyl-4-yl)-(4-(benzofuran-2-yl)-phenyl)-amine, 6.2 g of 4-bromo-(7-azabenzoxazol-2-yl)-benzene, 0.4 g of tris(dibenzylideneacetone)dipalladium(0), 0.2 g of tri-t-butylphosphine, and 3.0 g of sodium t-butoxide were placed in a nitrogen-purged reaction vessel and stirred in a toluene solvent overnight under reflux. The reaction system was allowed to cool and then filtered, and the filtrate was concentrated. Thus, a crude product was obtained. The obtained crude product was purified through crystallization using a toluene/acetone mixed solvent to obtain 8.6 g (with a yield of 61.0%) of an yellow powder of 4-(7-azabenzoxazol-2-yl)-phenyl-(4'-(naphthalen-1-yl)-biphenyl-4-yl)-(4-(benzofuran-2-yl)-phenyl)-amine: Compound (166).

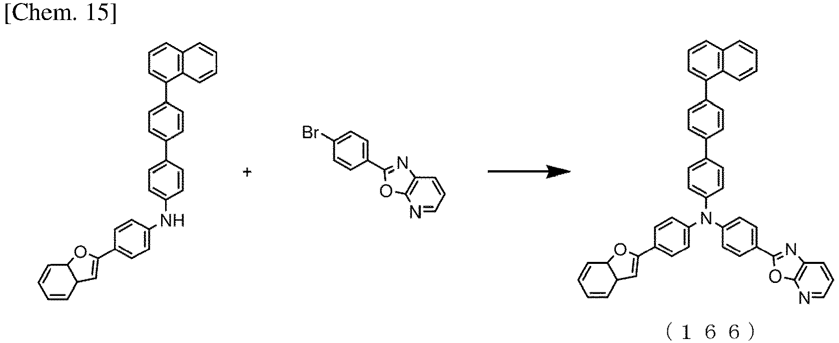

The structure of the obtained yellow powder was identified using NMR.

In ¹H-NMR (CDCl₃), the following signals of 31 hydrogens were detected.
δ (ppm) = 8.32 (1H), 8.18 (2H), 8.03 (1H), 7.99 (1H), 7.93 (1H), 7.89 (1H), 7.85 (2H), 7.75 (2H), 7.69 (2H), 7.61-7.58 (3H), 7.56-7.44 (5H), 7.35-7.22 (9H), 7.00 (1H).

### Example 9

### <Synthesis of 4-(7-azabenzoxazol-2-yl)-phenyl-(4'-(dibenzothiophen-4-yl)-biphenyl-4-yl)-(4-(benzofur an-2-yl)-phenyl)-amine: Compound (167)>

12.0 g of (4'-(dibenzothiophen-4-yl)-biphenyl-4-yl)-(4-(benzofuran-2-yl)-phenyl)-amine, 6.7 g of 4-bromo-(7-azabenzoxazol-2-yl)-benzene, 0.6 g of tris(dibenzylideneacetone)dipalladium(0), 0.5 g of tri-t-butylphosphine, and 3.2 g of sodium t-butoxide were placed in a nitrogen-purged reaction vessel and stirred in a toluene solvent overnight under reflux. The reaction system was allowed to cool and then filtered, and the filtrate was concentrated. Thus, a crude product was obtained. The obtained crude product was purified through crystallization using a toluene/acetone mixed solvent to obtain 9.5 g (with a yield of 58.0%) of an yellow powder of 4-(7-azabenzoxazol-2-yl)-phenyl-(4'-(dibenzothiophen-4-yl)-biphenyl-4-yl)-(4-(benzofur an-2-yl)-phenyl)-amine: Compound (167).

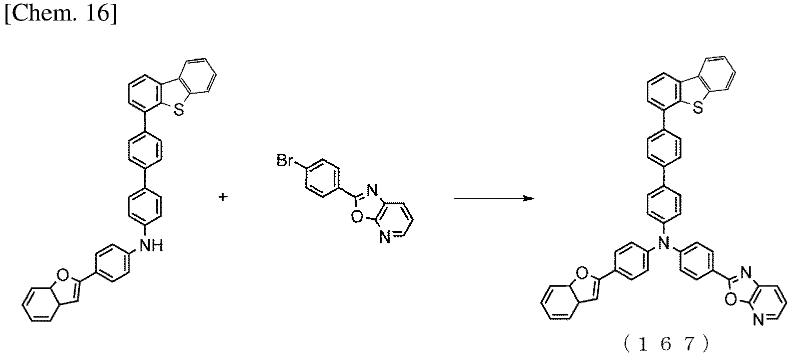

The structure of the obtained yellow powder was identified using NMR.

In ¹H-NMR (CDCl₃), the following signals of 31 hydrogens were detected.
δ (ppm) =8.31 (1H), 8.22-8.16(4H), 8.02 (1H), 7.85 (5H), 7.77 (2H), 7.68 (2H), 7.60-7.47 (6H), 7.34-7.22 (9H), 6.99 (1H).

### Example 10

### <Synthesis of 4-(7-azabenzoxazol-2-yl)-phenyl-(4-(dibenzothiophen-4-yl)-phenyl)-(4'-(benzofuran-2-yl )-biphenyl-4-yl)-amine: Compound (168)>

7.0 g of (4-(dibenzothiophen-4-yl)-phenyl)-(4' -(benzofuran-2-yl)-biphenyl-4-yl)-amine, 4.4 g of 4-bromo-(7-azabenzoxazol-2-yl)-benzene, 0.4 g of tris(dibenzylideneacetone)dipalladium(0), 0.3 g of tri-t-butylphosphine, and 1.9 g of sodium t-butoxide were placed in a nitrogen-purged reaction vessel and stirred in a toluene solvent overnight under reflux. The reaction system was allowed to cool and then dispersed and washed with methanol added thereto, and a solid was removed by filtration. Thus, a crude product was obtained. The obtained crude product was purified through crystallization using a monochlorobenzene/acetone mixed solvent to obtain 8.5 g (with a yield of 89.8%) of an yellow powder of 4-(7-azabenzoxazol-2-yl)-phenyl-(4-(dibenzothiophen-4-yl)-phenyl)-(4'-(benzofuran-2-yl )-biphenyl-4-yl)-amine: Compound (168).

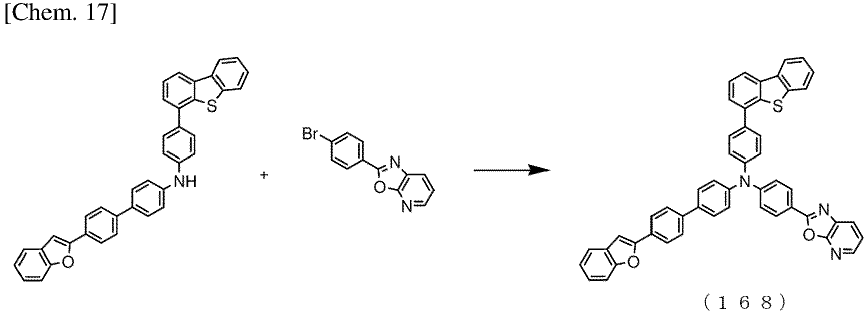

The structure of the obtained yellow powder was identified using NMR.

In ¹H-NMR (CDCl₃), the following signals of 31 hydrogens were detected.
δ (ppm) = 8.31 (1H), 8.21-8.16(4H), 8.03 (1H), 7.96 (2H), 7.86 (1H), 7.76-7.71 (4H), 7.67 (2H), 7.61-7.52 (4H), 7.48 (2H), 7.38-7.23 (9H), 7.08 (1H).

### Example 11

### <Synthesis of 4'-(7-azabenzoxazol-2-yl)-biphenyl-4-yl-(4-naphthalen-2-yl-phenyl)-(4-phenanthren-9-yl -phenyl)-amine: Compound (172)>

6.0 g of (4-(naphthalen-2-yl-phenyl)-(4-(phenanthren-9-yl-phenyl)amine, 4.3 g of 2-(4'-chloro-biphenyl-4-yl)-7-azabenzoxazole, 0.4 g of tris(dibenzylideneacetone)dipalladium(0), 0.3 g of tri-t-butylphosphine, and 1.8 g of sodium t-butoxide were placed in a nitrogen-purged reaction vessel and stirred in a toluene solvent overnight under reflux. The reaction system was allowed to cool and then dispersed and washed with methanol added thereto, and a solid was removed by filtration. Thus, a crude product was obtained. The obtained crude product was purified through crystallization using a monochlorobenzene/acetone mixed solvent to obtain 5.1 g (with a yield of 54.0%) of an yellow powder of 4'-(7-azabenzoxazol-2-yl)-biphenyl-4-yl-(4-naphthalen-2-yl-phenyl)-(4-phenanthren-9-yl -phenyl)-amine: Compound (172).

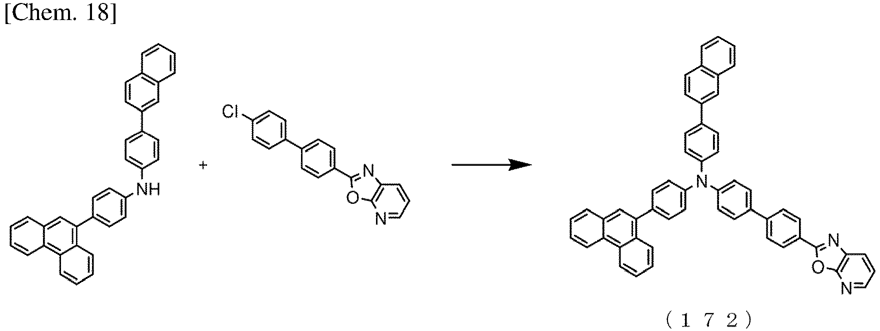

The structure of the obtained yellow powder was identified using NMR.

In ¹H-NMR (CDCl₃), the following signals of 35 hydrogens were detected. δ (ppm) = 8.80 (1H), 8.74 (1H), 8.38-8.36 (3H), 8.09-8.07 (3H), 7.94-7.86 (4H), 7.82-7.59 (12H), 7.53-7.46 (4H), 7.39-7.35 (7H).

### Example 12

### <Synthesis of 4'-(7-azabenzoxazol-2-yl)-biphenyl-4-yl-(4-dibenzofuran-3-yl-phenyl)-(4-naphthalen-2-y l-phenyl)-amine: Compound (173)>

6.0 g of (4-dibenzofuran-3-yl-phenyl)-(4-naphthalen-2-yl-phenyl)amine, 4.4 g of 2-(4'-chloro-biphenyl-4-yl)-7-azabenzoxazole, 0.4 g of tris(dibenzylideneacetone)dipalladium(0), 0.3 g of tri-t-butylphosphine, and 1.9 g of sodium t-butoxide were placed in a nitrogen-purged reaction vessel and stirred in a xylene solvent overnight under reflux. The reaction system was allowed to cool and then dispersed and washed with methanol added thereto, and a solid was removed by filtration. Thus, a crude product was obtained. The obtained crude product was purified through crystallization using a toluene/acetone mixed solvent to obtain 2.5 g (with a yield of 26.3%) of an yellow powder of 4'-(7-azabenzoxazol-2-yl)-biphenyl-4-yl-(4-dibenzofuran-3-yl-phenyl)-(4-naphthalen-2-y l-phenyl)-amine: Compound (173).

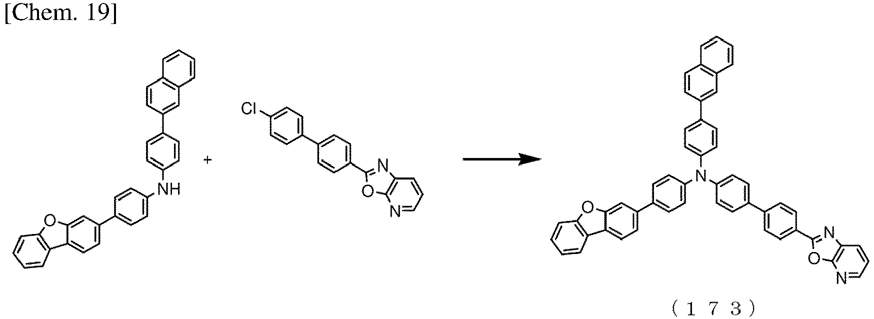

The structure of the obtained yellow powder was identified using NMR.

In ¹H-NMR (CDCl₃), the following signals of 33 hydrogens were detected. δ (ppm) = 8.37-8.35 (3H), 8.09-8.06 (2H), 8.01-7.86 (5H), 7.81-7.76 (4H), 7.71-7.58 (8H), 7.53-7.45 (3H), 7.38-7.31 (8H).

### Example 13

The melting point and the glass transition point of the compounds obtained in the examples were measured using a high-sensitivity differential scanning calorimeter (DSC3100SA manufactured by Bruker AXS K.K.). Table 1 collectively shows the measurement results.

**Table 1**

| | Melting point | Glass transition point |
|---|---|---|
| Compound (123) | - | 123.1°C |
| Compound (124) | - | 120.4°C |
| Compound (25) | 112.6°C | 108.8°C |
| Compound (44) | 249.0°C | 105.1°C |
| Compound (140) | 251.1°C | 124.8°C |
| Compound (144) | 291.6°C | 144.0°C |
| Compound (146) | 318.2°C | 159.1°C |
| Compound (166) | - | 125.4°C |
| Compound (167) | - | 138.6°C |
| Compound (168) | - | 136.7°C |
| Compound (172) | - | 138.0°C |
| Compound (173) | - | 125.9°C |

It is seen from the results that the compounds obtained in the examples had a glass transition point of 100°C or higher, which indicates that these compounds are stable in a thin film state.

### Example 14

A vapor-deposited film with a film thickness of 80 nm was formed on a silicon substrate using each of the compounds obtained in the examples, and the refractive index n and the extinction coefficient k at wavelengths of 400 nm and 410 nm were measured using a spectroscopic measurement device (F10-RT-UV manufactured by Filmetrics). For comparison, the measurement was also performed on Comparative Compound (2-1) having the structural formula below and Alq₃ (see Patent Literature 4, for example). Table 2 collectively shows the measurement results.

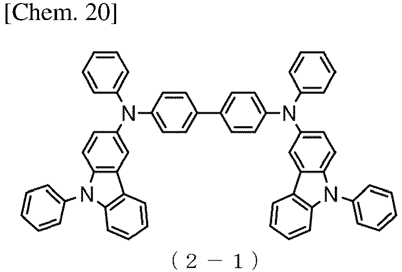

**Table 2**

| | Refraction index n (λ: 450 nm) | Refraction index n (λ: 750 nm) | Extinction coefficient k (λ: 400 nm) | Extinction coefficient k (λ: 410 nm) |
|---|---|---|---|---|
| Compound (123) | 2.29 | 1.89 | 0.60 | 0.53 |
| Compound (124) | 2.43 | 1.91 | 0.80 | 0.71 |
| Compound (25) | 2.34 | 1.89 | 0.85 | 0.70 |
| Compound (44) | 2.46 | 1.91 | 1.00 | 0.83 |
| Compound (140) | 2.52 | 1.91 | 1.00 | 0.98 |
| Compound (144) | 2.44 | 1.91 | 0.89 | 0.84 |
| Compound (146) | 2.40 | 1.89 | 1.02 | 0.85 |
| Compound (166) | 2.48 | 1.89 | 1.08 | 0.89 |
| Compound (167) | 2.45 | 1.92 | 0.98 | 0.80 |
| Compound (168) | 2.47 | 1.94 | 0.95 | 0.78 |
| Compound (172) | 2.45 | 1.94 | 0.76 | 0.67 |
| Compound (173) | 2.47 | 1.95 | 0.90 | 0.75 |
| Alq₃ | 1.88 | 1.73 | 0.16 | 0.14 |
| Compound (2-1) | 1.93 | 1.78 | 0.15 | 0.06 |

As shown in Table 2, the refractive indices at a wavelength of 450 nm of Comparative Compound (2-1) and Alq₃ ranged from 1.88 to 1.93, whereas those of the compounds of the present invention ranged from 2.29 to 2.52. Also, the refractive indices at a wavelength of 750 nm of Comparative Compound (2-1) and Alq₃ ranged from 1.73 to 1.78, whereas those of the compounds of the present invention ranged from 1.89 to 1.95. This indicates that the use of any of the compounds of the present invention in an organic EL element can be expected to improve the light extraction efficiency of the organic EL element.

The extinction coefficients at wavelengths of 400 to 410 nm of Comparative Compound (2-1) and Alq₃ ranged from 0.06 to 0.16, whereas those of the compounds of the present invention ranged from 0.53 to 1.08. This indicates that each compound of the present invention absorbs light rays with wavelengths of 400 to 410 nm of sunlight well and, when used as a capping layer of an organic EL element, can suppress the effect of those light rays on the materials inside the element.

### Example 15

With use of each compound of the present invention, a toluene solution with a concentration of 10⁻⁵ mol/L was prepared, and the absorbance at wavelengths of 400 nm and 410 nm was measured using an UV-Visible/NIR spectrophotometer (V-650 manufactured by JASCO Corporation). Also, toluene solutions with four different concentrations of 5×10⁻⁶ mol/L, 1×10⁻⁵ mol/L, 1.5×10⁻⁵ mol/L, and 2.0×10⁻⁵ mol/L were prepared and subjected to measurements using a UV-NIR spectrophotometer (V-650 manufactured by JASCO Corporation) to obtain a calibration curve, and the absorption coefficient was calculated based on the obtained calibration curve. For comparison, the absorption coefficients of Comparative Compound (2-1) having the structural formula above and Alq₃ were also calculated by measuring the absorbance in a similar manner. Table 3 collectively shows the results.

**Table 3**

| | Peak wavelength (λ max) | Absorbance (λ: 400 nm) | Absorbance (λ: 410 nm) | Absorption coefficient |
|---|---|---|---|---|
| Compound (123) | 379 nm | 0.81 | 0.53 | 110026 |
| Compound (124) | 363 nm | 0.98 | 0.67 | 133181 |
| Compound (25) | 382 nm | 0.81 | 0.43 | 113106 |
| Compound (44) | 381 nm | 0.59 | 0.36 | 73952 |
| Compound (140) | 397 nm | 1.28 | 0.90 | 129622 |
| Compound (144) | 395 nm | 1.12 | 0.71 | 116298 |
| Compound (146) | 392 nm | 1.35 | 0.55 | 153646 |
| Compound (166) | 366 nm | 0.83 | 0.56 | 105338 |
| Compound (167) | 369 nm | 0.85 | 0.57 | 111874 |
| Compound (168) | 363 nm | 0.72 | 0.43 | 101901 |
| Compound (172) | 362 nm | 0.59 | 0.41 | 82905 |
| Compound (173) | 370 nm | 0.82 | 0.58 | 127727 |
| Alq₃ | 394 nm | 0.07 | 0.06 | 7518 |
| Compound (2-1) | 358 nm | 0.07 | 0.02 | 48856 |

As shown in Table 3, with regard to the absorbance at wavelengths of 400 to 410 nm, the values of the absorbance at wavelengths of 400 to 410 nm of Comparative Compound (2-1) and Alq₃ ranged from 0.02 to 0.07, whereas those of the compounds of the present invention were as high as from 0.36 to 1.35. This indicates that the compounds of the present invention can absorb light rays with wavelengths of 400 to 410 nm of sunlight well. In addition, with regard to the absorption coefficient, the compounds of the present invention had values of 70000 or more, which are greater than the numerical value of the absorption coefficients of the comparative compounds. This means that the compounds of the present invention absorb light better than the comparative compounds under the same concentration conditions.

### Example 16

An organic EL element was prepared using Compound (123) obtained in Example 1, and the properties thereof were measured in the atmosphere at normal temperature.

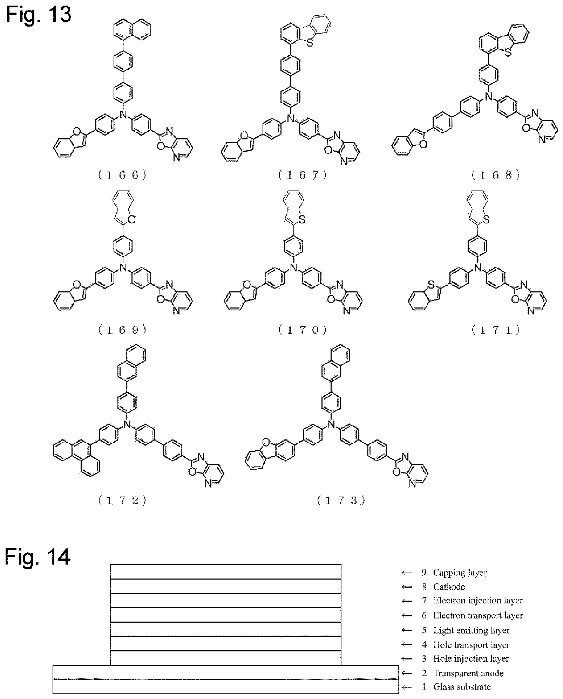

As shown in Fig. 14, the organic EL element was prepared by forming a reflective ITO electrode serving as a transparent anode 2 on a glass substrate 1 in advance, and furthermore, vapor-depositing a hole injection layer 3, a hole transport layer 4, a light emitting layer 5, an electron transport layer 6, an electron injection layer 7, a cathode 8, and a capping layer 9 in this order on the ITO electrode.

Specifically, a glass substrate 1 on which an ITO film with a thickness of 50 nm, a reflective film of silver alloy with a film thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 2 minutes. Then, the glass substrate with ITO was attached inside a vacuum vapor deposition machine, and the pressure was reduced to 0.001 Pa or less. Subsequently, an electron acceptor (Acceptor-1) having the structural formula below and Compound (3-1) having the structural formula below were vapor-deposited so as to cover the transparent anode 2 through binary vapor deposition at such vapor deposition rates that the ratio of the vapor deposition rate of Acceptor-1 to the vapor deposition rate of Compound (3-1) was 3:97, and a film with a thickness of 10 nm was thus formed as a hole injection layer 3.

A film of Compound (3-1) having the structural formula below was formed on the hole injection layer 3 as a hole transport layer 4 with a film thickness of 140 nm.

Compound (3-2) having the structural formula below and Compound (3-3) having the structural formula below were vapor-deposited on the hole transport layer 4 through binary vapor deposition at such vapor deposition rates that the ratio of the vapor deposition rate of (3-2) to the vapor deposition rate of (3-3) was 5:95, and a film with a thickness of 20 nm was thus formed as a light emitting layer 5.

Compound (3-4) having the structural formula below and Compound (3-5) having the structural formula below were vapor-deposited on the light emitting layer 5 through binary vapor deposition at such vapor deposition rates that the ratio of the vapor deposition rate of (3-4) to the vapor deposition rate of (3-5) was 50:50, and a film with a thickness of 30 nm was thus formed as an electron transport layer 6.

A film of lithium fluoride was formed on the electron transport layer 6, as an electron injection layer 7 with a thickness of 1 nm.

A film of a magnesium-silver alloy was formed on the electron injection layer 7, as a cathode 8 with a film thickness of 12 nm.

Lastly, a film of Compound (123) of Example 1 was formed as a capping layer 9 with a film thickness of 60 nm.

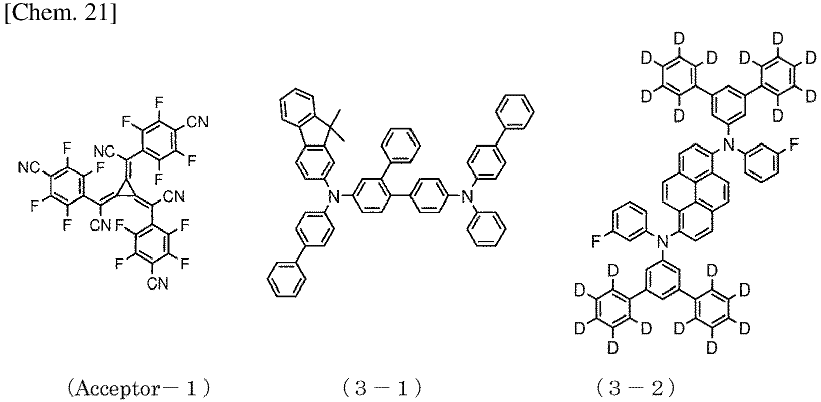

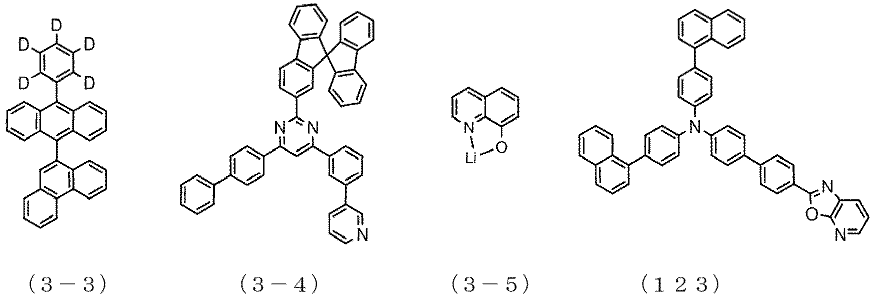

### Example 17

An organic EL element was prepared under similar conditions to those of Example 16, except that Compound (124) of Example 2 was used instead of Compound (123) of Example 1 to form a film with a thickness of 60 nm as the capping layer 9. The properties of the prepared organic EL element were measured in the atmosphere at normal temperature.

### Example 18

An organic EL element was prepared under similar conditions to those of Example 16, except that Compound (25) of Example 3 was used instead of Compound (123) of Example 1 to form a film with a thickness of 60 nm as the capping layer 9. The properties of the prepared organic EL element were measured in the atmosphere at normal temperature.

### Example 19

An organic EL element was prepared under similar conditions to those of Example 16, except that Compound (44) of Example 4 was used instead of Compound (123) of Example 1 to form a film with a thickness of 60 nm as the capping layer 9. The properties of the prepared organic EL element were measured in the atmosphere at normal temperature.

### Example 20

An organic EL element was prepared under similar conditions to those of Example 16, except that Compound (140) of Example 5 was used instead of Compound (123) of Example 1 to form a film with a thickness of 60 nm as the capping layer 9. The properties of the prepared organic EL element were measured in the atmosphere at normal temperature.

### Example 21

An organic EL element was prepared under similar conditions to those of Example 16, except that Compound (144) of Example 6 was used instead of Compound (123) of Example 1 to form a film with a thickness of 60 nm as the capping layer 9. The properties of the prepared organic EL element were measured in the atmosphere at normal temperature.

### Example 22

An organic EL element was prepared under similar conditions to those of Example 16, except that Compound (146) of Example 7 was used instead of Compound (123) of Example 1 to form a film with a thickness of 60 nm as the capping layer 9. The properties of the prepared organic EL element were measured in the atmosphere at normal temperature.

### Example 23

An organic EL element was prepared under similar conditions to those of Example 16, except that Compound (166) of Example 8 was used instead of Compound (123) of Example 1 to form a film with a thickness of 60 nm as the capping layer 9. The properties of the prepared organic EL element were measured in the atmosphere at normal temperature.

### Example 24

An organic EL element was prepared under similar conditions to those of Example 16, except that Compound (167) of Example 9 was used instead of Compound (123) of Example 1 to form a film with a thickness of 60 nm as the capping layer 9. The properties of the prepared organic EL element were measured in the atmosphere at normal temperature.

### Example 25

An organic EL element was prepared under similar conditions to those of Example 16, except that Compound (168) of Example 10 was used instead of Compound (123) of Example 1 to form a film with a thickness of 60 nm as the capping layer 9. The properties of the prepared organic EL element were measured in the atmosphere at normal temperature. Table 4 collectively shows the measurement results of light emission properties that were obtained when a DC voltage was applied to the prepared organic EL elements.

### Example 26

An organic EL element was prepared under similar conditions to those of Example 16, except that Compound (172) of Example 11 was used instead of Compound (123) of Example 1 to form a film with a thickness of 60 nm as the capping layer 9. The properties of the prepared organic EL element were measured in the atmosphere at normal temperature.

### Example 27

An organic EL element was prepared under similar conditions to those of Example 16, except that Compound (173) of Example 12 was used instead of Compound (123) of Example 1 to form a film with a thickness of 60 nm as the capping layer 9. The properties of the prepared organic EL element were measured in the atmosphere at normal temperature.

### Comparative Example 1

For comparison, an organic EL element was prepared under similar conditions to those of Example 16, except that Alq₃ was used instead of Compound (123) of Example 1 to form a film with a thickness of 60 nm as the capping layer 9. The properties of the prepared organic EL element were measured in the atmosphere at normal temperature.

### Comparative Example 2

For comparison, an organic EL element was prepared under similar conditions to those of Example 16, except that Compound (2-1) having the structural formula above was used instead of Compound (123) of Example 1 to form a film with a thickness of 60 nm as the capping layer 9. The properties of the prepared organic EL element were measured in the atmosphere at normal temperature.

With use of the organic EL elements prepared in Examples 16 to 27 and Comparative Examples 1 and 2, the voltage, the luminance, the light emission efficiency, the power efficiency, and the element lifespan were measured. Table 4 collectively shows the results. The voltage, the luminance, the light emission efficiency, and the power efficiency were measured by performing constant current driving at 10 mA/cm². The element lifespan was defined as follows: when constant current driving was performed at 10 mA/cm² with the initial luminance being set to 100%, the time taken for the luminance to decay to 95% of the initial luminance was measured as the element lifespan.

**Table 4**

| | Capping layer | Voltage (V) | Luminance (cd/A) | Light emission efficiency (cd/A) | Power efficiency (lm/W) | Element lifespan to decay of 95% |
|---|---|---|---|---|---|---|
| Ex. 16 | Compound (123) | 3.66 | 824 | 8.24 | 7.07 | 166 h |
| Ex. 17 | Compound (124) | 3.67 | 838 | 8.37 | 7.16 | 157 h |
| Ex. 18 | Compound (25) | 3.66 | 826 | 8.26 | 7.10 | 168 h |
| Ex. 19 | Compound (44) | 3.66 | 839 | 8.39 | 7.21 | 158 h |
| Ex. 20 | Compound (140) | 3.67 | 852 | 8.52 | 7.30 | 140 h |
| Ex. 21 | Compound (144) | 3.65 | 842 | 8.42 | 7.26 | 160 h |
| Ex. 22 | Compound (146) | 3.67 | 833 | 8.33 | 7.13 | 151 h |
| Ex. 23 | Compound (166) | 3.63 | 848 | 8.48 | 7.34 | 158 h |
| Ex. 24 | Compound (167) | 3.64 | 843 | 8.43 | 7.28 | 147 h |
| Ex. 25 | Compound (168) | 3.66 | 846 | 8.46 | 7.26 | 146 h |
| Ex. 26 | Compound (172) | 3.65 | 844 | 8.44 | 7.27 | 150 h |
| Ex. 27 | Compound (173) | 3.67 | 845 | 8.45 | 7.23 | 146 h |
| Com. Ex. 1 | Alq₃ | 3.65 | 714 | 7.14 | 6.15 | 114 h |
| Com. Ex. 2 | Compound (2-1) | 3.66 | 735 | 7.34 | 6.30 | 133 h |

As shown in Table 4, while the elements of Comparative Examples 1 and 2 and those of Examples 16 to 27 had substantially similar driving voltages at a current density of 10 mA/cm², the elements of Examples 16 to 27 exhibited marked improvements in the luminance, the light emission efficiency, the power efficiency, and the lifespan compared with the elements of Comparative Examples 1 and 2. This indicates that, when a material that has a high refractive index and is suitable for use in the organic EL device of the present invention is contained in the capping layer, the light extraction efficiency can be significantly improved.

### Industrial Applicability

An amine compound having an azabenzazole ring structure and being represented by the general formula (a-1) of the present invention has a high absorption coefficient and a high refractive index, can significantly improve the light extraction efficiency, and is stable in a thin film state, and thus, it is excellent as a compound suitable for use in an organic EL element. An organic EL element that is produced using this compound can achieve good efficiency and also absorb sunlight, thereby preventing sunlight from affecting materials inside the element, and can therefore achieve improved durability and light resistance. Furthermore, since the compound does not have absorption in blue, green, and red wavelength regions, the organic EL element in which the compound is used provides good color purity and sharpness and is therefore particularly suitably used when it is desired to display a bright image. Thus, for example, the organic EL element can be applied to applications such as home electric appliances and lighting equipment.

### List of Reference Numerals

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode
- 9: Capping layer

## Claims

1. An amine compound having an azabenzoxazole ring structure and being represented by the general formula (a-1) below: 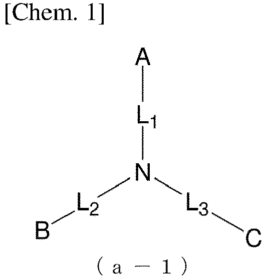
wherein A, B, and C may be the same or different, and each represents a group represented by the general formula (b-1) below, a substituted or unsubstituted aromatic hydrocarbon group, a substituted or unsubstituted aromatic heterocyclic group, or a substituted or unsubstituted fused polycyclic aromatic group, however, at least one of A, B, and C representing a group represented by the general formula (b-1) below, and
L₁, L₂, and L₃ may be the same or different, and each represents a single bond, a substituted or unsubstituted divalent aromatic hydrocarbon group, a substituted or unsubstituted divalent aromatic heterocyclic group, or a substituted or unsubstituted divalent fused polycyclic aromatic group: 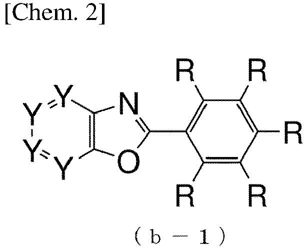
wherein symbols R may be the same or different, and each represents a binding site for L₁, L₂, or L₃ in the general formula (a-1), a hydrogen atom, a deuterium atom, a chlorine atom, a cyano group, a nitro group, a trimethylsilyl group, a triphenylsilyl group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aryloxy group, a substituted or unsubstituted aromatic hydrocarbon group, a substituted or unsubstituted aromatic heterocyclic group, or a substituted or unsubstituted fused polycyclic aromatic group, and one of the symbols R represents a binding site for L₁, L₂, or L₃ in the general formula (a-1), and
symbols Y may be the same or different, and each represents a carbon atom or nitrogen atom substituted by R, however at least one of the symbols Y representing a nitrogen atom substituted by R.

2. The amine compound having an azabenzoxazole ring structure according to claim 1,
wherein among of A, B, and C in the general formula (a-1), a group other than the group represented by the general formula (b-1), is a substituted or unsubstituted phenyl group, a substituted or unsubstituted biphenyl group, a substituted or unsubstituted naphthyl group, a substituted or unsubstituted phenanthrenyl group, a substituted or unsubstituted carbazolyl group, a substituted or unsubstituted benzofuranyl group, a substituted or unsubstituted benzothiophenyl group, a substituted or unsubstituted indolyl group, a substituted or unsubstituted benzimidazolyl group, a substituted or unsubstituted imidazopyridyl group, a substituted or unsubstituted benzoxazolyl group, a substituted or unsubstituted benzothiazolyl group, a substituted or unsubstituted dibenzofuranyl group, or a substituted or unsubstituted dibenzothiophenyl group.

3. The amine compound having an azabenzoxazole ring structure according to claim 1, wherein the group represented by the general formula (b-1) is a group represented by the general formula (b-2) or (b-3) below: 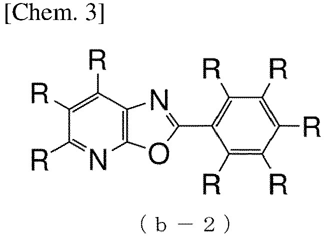 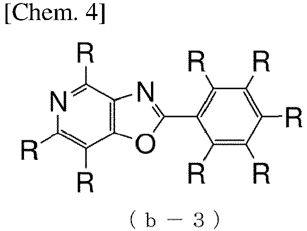 wherein symbols R are the same as defined for the symbols R in the general formula (b-1).

4. The amine having an azabenzoxazole ring structure according to claim 3, wherein the group represented by the general formula (b-2) is a group represented by the general formula (b-4) below, and the group represented by the general formula (b-3) is a group represented by the general formula (b-5) below: 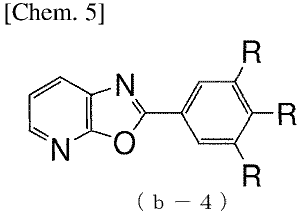 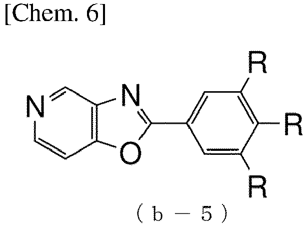 wherein symbols R are the same as defined for the symbols R in the general formula (b-1).

5. The amine compound having an azabenzoxazole ring structure according to claim 1, wherein L₁, L₂, and L₃ in the general formula (a-1) each represents a single bond, a substituted or unsubstituted phenylene group, a substituted or unsubstituted biphenylene group, a substituted or unsubstituted naphthylene group, a substituted or unsubstituted dibenzofuranylene group, or a substituted or unsubstituted dibenzothiophenylene group.

6. The amine compound having an azabenzoxazole ring structure according to claim 4, wherein only one of A, B, and C in the general formula (a-1) represents a group represented by the general formula (b-4) or (b-5).

7. The amine compound having an azabenzoxazole ring structure according to claim 4, wherein two of A, B, and C in the general formula (a-1) each represents a group represented by the general formula (b-4) or (b-5).

8. The amine compound having an azabenzoxazole ring structure according to claim 4, wherein all of A, B, and C in the general formula (a-1) each represents a group represented by the general formula (b-4) or (b-5).

9. An organic EL element comprising at least an anode electrode, a hole transport layer, a light emitting layer, an electron transport layer, a cathode electrode, and a capping layer arranged in this order, wherein the capping layer contains the amine compound having an azabenzoxazole ring structure according to any one of claims 1 to 8.

10. The organic EL element according to claim 9, wherein the capping layer has an extinction coefficient of 0.2 or more in a wavelength range of 400 to 410 nm, and an absorbance of 0.2 or more in a wavelength range of 400 to 410 nm in an absorption spectrum at a concentration of 10⁻⁵mol/L.

11. The organic EL element according to claim 9, wherein the capping layer has a refractive index of 1.85 or more in a wavelength range of 450 to 750 nm.

12. The organic EL element according to claim 9,
wherein the capping layer is
a mixed layer composed of two or more compounds including the said amine compound having an azabenzoxazole ring structure, or
a lamination layer with two or more layers wherein each layer contains the said amine compound alone respectively.

13. An electronic element having a pair of electrodes and an organic layer sandwiched therebetween, wherein the organic layer contains the amine compound having an azabenzoxazole ring structure according to any one of claims 1 to 8.

14. An electronic device in which the electronic element according to claim 13 is used.
